# EUROPEAN PATENT APPLICATION

(11) **EP 3 196 954 A1**
(43) Date of publication of application: **26.07.2017**
(21) Application number: 15841702.2
(22) Date of filing: 26.08.2015
(51) Int. Cl.: H01L 51/50, C09K 11/06, H05B 33/10

(54) **ORGANIC ELECTROLUMINESCENT ELEMENT**

(30) Priority: 17.09.2014 JP 2014188593
(71) Applicant: NIPPON STEEL & SUMIKIN CHEMICAL CO., LTD., Chiyoda-ku, Tokyo 101-0021 (JP)
(72) Inventor: TADA Masashi, Kitakyushu-shi, Fukuoka 804-8503 (JP); KAI Takahiro, Kitakyushu-shi, Fukuoka 804-8503 (JP); HOTTA Masanori, Kitakyushu-shi, Fukuoka 804-8503 (JP); OGAWA Junya, Kitakyushu-shi, Fukuoka 804-8503 (JP); SAKAI Mitsuru, Kitakyushu-shi, Fukuoka 804-8503 (JP); IKENAGA Yuji, Kitakyushu-shi, Fukuoka 804-8503 (JP); UEDA Tokiko, Kitakyushu-shi, Fukuoka 804-8503 (JP); NOGUCHI Katsuhide, Kitakyushu-shi, Fukuoka 804-8503 (JP)
(74) Representative: Vossius & Partner Patentanwälte Rechtsanwälte mbB
(86) International application number: PCT/JP2015/074050
(87) International publication number: WO 2016/042997

(57) **Abstract**

Provided is a homogeneous organic EL device having a low driving voltage, high luminous efficiency, and a long lifetime. The device is an organic electroluminescent device including a light-emitting layer between an anode and a cathode opposite to each other, in which: the light-emitting layer contains a host material and a light-emitting dopant material; the host material is a material obtained by preliminarily mixing two or more kinds of compounds selected from compounds each having a structure in which two nitrogen atoms of an indolocarbazole ring are each substituted with an aromatic hydrocarbon group or an aromatic heterocyclic group; and the light-emitting layer is formed by co-depositing the preliminarily mixed host material and the light-emitting dopant material in a vacuum.

## Description

### Technical Field

The present invention relates to an organic electroluminescent device (referred to as "organic EL device").

### Background Art

In general, the simplest structure of an organic EL device includes a light-emitting layer and a pair of opposing electrodes between which the layer is interposed. That is, in the organic EL device, when an electric field is applied between both the electrodes, an electron is injected from a cathode and a hole is injected from an anode. The device utilizes a phenomenon in which energy produced at the time of the recombination of the electron and the hole in the light-emitting layer is emitted as light.

In recent years, progress has been made in developing an organic EL device using an organic thin film. In particular, development has been made in order to enhance luminous efficiency. In the development, the efficiency of injection of carriers from electrodes has been improved through optimization of the kind of the electrodes. In addition, there has been developed a device using a hole-transporting layer formed of an aromatic diamine and a light-emitting layer also serving as an electron-transporting layer formed of an 8-hydroxyquinoline aluminum complex (hereinafter referred to as Alq3), resulting in a significant improvement in luminous efficiency, as compared to related-art devices. Thus, the development of the organic EL device has been promoted in order to accomplish its practical application to a high-performance flat panel having features such as self-luminescence and rapid response.

Investigations have been made also on using a phosphorescent light-emitting material rather than a fluorescent light-emitting material as an attempt to improve the luminous efficiency of a device. Many kinds of devices including the device in which a hole-transporting layer formed of an aromatic diamine and a light-emitting layer formed of Alq3 are formed emit light by using fluorescent light emission. However, by using phosphorescent light emission (light emission from a triplet excited state), luminous efficiency is expected to be improved by from about three times to four times, as compared to the case of using the related-art devices in which fluorescent light (light emission from a singlet excited state) is used. In order to accomplish this purpose, investigations have been made on using a coumarin derivative or a benzophenone derivative in a light-emitting layer, but extremely low luminance has only been provided. Then, investigations have been made on using a europium complex as an attempt to use a triplet excited state, but highly efficient light emission has not been accomplished. As a phosphorescent light-emitting dopant to be used for the phosphorescent light emission, many investigations have been made mainly on an organometallic complex, such as an iridium complex as disclosed in Patent Literature 1, and some materials having high luminous efficiency have been found. However, a further improvement has been desired in terms of durability.

Further, a high-efficiency organic EL device utilizing delayed fluorescence has been recently developed. In, for example, Patent Literature 2, there is a disclosure of an organic EL device utilizing a triplet-triplet fusion (TTF) mechanism serving as one of the delayed fluorescence mechanisms. The TTF mechanism utilizes a phenomenon in which a singlet exciton is produced by collision between two triplet excitons, and is considered to be capable of improving internal quantum efficiency to 40% in theory. However, a further improvement in efficiency has been required because the efficiency of the device is lower than that of a phosphorescent light-emitting organic EL device.

Meanwhile, in Patent Literature 3, there is a disclosure of an organic EL device utilizing a thermally activated delayed fluorescence (TADF) mechanism. The TADF mechanism utilizes a phenomenon in which inverse intersystem crossing from a triplet exciton to a singlet exciton occurs in a material having a small energy difference between a singlet level and a triplet level, and is considered to be capable of improving internal quantum efficiency to 100% in theory. However, a further improvement in durability has been required as in a phosphorescent device.

### Citation List

### Patent Literature

[PTL 1] WO 2001/041512 A
[PTL 2] WO 2010/134350 A
[PTL 3] WO 2011/070963 A
[PTL 4] WO 2008/056745 A
[PTL 5] WO 2010/098246 A
[PTL 6] WO 2012/087955 A
[PTL 7] WO 2011/136755 A

In Patent Literature 4, there is a disclosure of the use of an indolocarbazole compound as a host material. In addition, in each of Patent Literatures 5 and 6, there is a disclosure of the use of two or more kinds of indolocarbazole compounds as a mixed host. Further, in Patent Literature 7, there is a disclosure of the use of preliminarily mixed indolocarbazole compounds as a host material.

However, none of the literatures teaches that a light-emitting layer is produced by using a host material, which is obtained by preliminarily mixing two or more kinds of indolocarbazole compounds, through vapor deposition.

### Summary of Invention

In order to apply an organic EL device to a display device, such as a flat panel display, or a light source, the luminous efficiency of the device needs to be improved, and at the same time, stability at the time of its driving needs to be sufficiently secured. In view of the above-mentioned present circumstances, an object of the present invention is to provide a practically useful organic EL device having high efficiency and high driving stability while having a low driving voltage with good reproducibility by depositing two or more kinds of host materials in a vacuum from one deposition source.

According to one embodiment of the present invention, there is provided an organic electroluminescent device, including one or more light-emitting layers between an anode and a cathode opposite to each other, in which at least one of the light-emitting layers produced by vacuum deposition contains a host material, which is obtained by preliminarily mixing two or more kinds of compounds selected from compounds each represented by the following general formula (1), and a light-emitting dopant material: 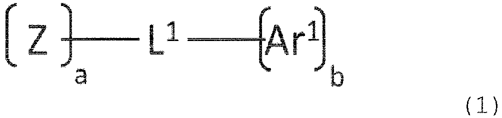 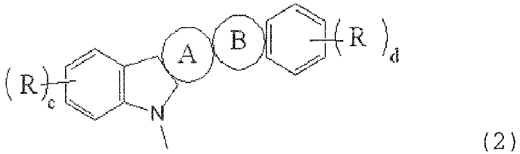 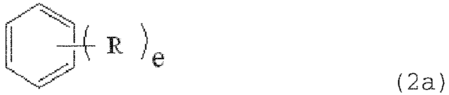 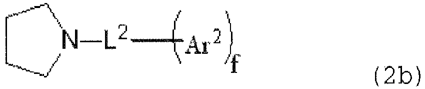 where:
Z represents a group represented by the general formula (2), a ring A represents an aromatic hydrocarbon ring represented by the formula (2a), a ring B represents a heterocycle represented by the formula (2b), and the ring A and the ring B are each fused with an adjacent ring;
L¹ and L² each independently represent an aromatic hydrocarbon group having 6 to 30 carbon atoms, or an aromatic heterocyclic group having 3 to 18 carbon atoms, and Ar¹ and Ar² each independently represent an aromatic hydrocarbon group having 6 to 30 carbon atoms, an aromatic heterocyclic group having 3 to 18 carbon atoms, or a linked aromatic group obtained by linking 2 to 6 of the groups;
R's each independently represent a cyano group, an aliphatic hydrocarbon group having 1 to 10 carbon atoms, a diarylamino group having 12 to 44 carbon atoms, a diarylboryl group having 12 to 44 carbon atoms, an aromatic hydrocarbon group having 6 to 18 carbon atoms, or an aromatic heterocyclic group having 3 to 18 carbon atoms; and
a represents an integer of from 1 to 3, b represents an integer of from 0 to 3, c and d each independently represent an integer of from 0 to 4, e represents an integer of from 0 to 2, and f represents an integer of from 0 to 3,
provided that L¹, L², Ar¹, Ar², and R's are each free from representing an aromatic heterocyclic group having an indolocarbazole ring represented by the general formula (2).

In the organic electroluminescent device, it is preferred that only one of L¹ and L² of at least one compound out of the compounds each represented by the general formula (1) represent a nitrogen-containing aromatic heterocyclic group having 3 to 18 carbon atoms, or a difference in evaporation temperature between the two or more kinds of compounds in the preliminarily mixed host material be 20°C or less.

The light-emitting dopant material may be a phosphorescent light-emitting dopant material, a fluorescent light-emitting dopant material, or a thermally activated delayed fluorescent light-emitting dopant material, and the phosphorescent light-emitting dopant material is preferably a phosphorescent light-emitting dopant material formed of an organometallic complex containing at least one metal selected from ruthenium, rhodium, palladium, silver, rhenium, osmium, iridium, platinum, and gold.

According to another embodiment of the present invention, there is provided a method of producing the organic electroluminescent device including the light-emitting layer, including depositing a material containing the host material ; which is obtained by preliminarily mixing the two or more kinds of compounds selected from the compounds each represented by the general formula (1), and the light-emitting dopant material in a vacuum to produce at least one light-emitting layer.

A light-emitting layer of the organic EL device of the present invention is obtained by mixing a plurality of host materials formed of a combination of specific host materials in advance, and depositing the materials, and hence the device can be a homogeneous organic EL device having a low driving voltage, high luminous efficiency, and a long lifetime.

### Brief Description of Drawings

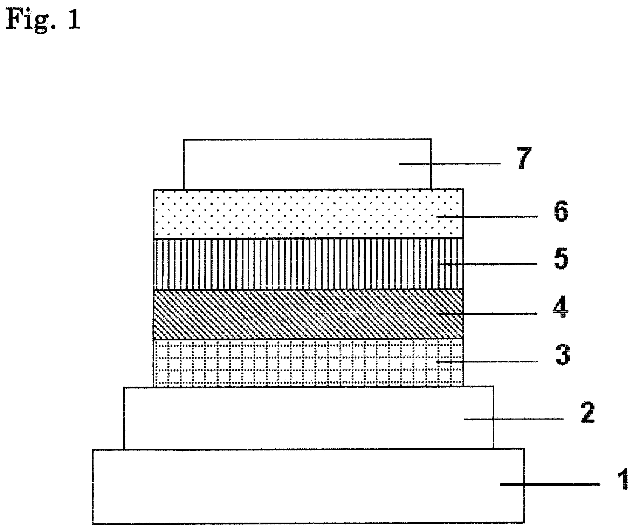

FIG. 1 is a schematic sectional view for illustrating an example of an organic EL device.

### Description of Embodiments

An organic EL device of the present invention includes one or more light-emitting layers between an anode and a cathode opposite to each other, and at least one layer of the light-emitting layers is produced by vacuum deposition and contains a host material, which is obtained by preliminarily mixing two or more kinds of compounds selected from compounds each represented by the general formula (1), and a light-emitting dopant material. The organic EL device includes an organic layer formed of a plurality of layers between the anode and the cathode opposite to each other. At least one layer of the plurality of layers is a light-emitting layer, and the number of the light-emitting layers may be two or more. The light-emitting layer (at least one light-emitting layer when the device includes a plurality of light-emitting layers) contains the host material and the light-emitting dopant material, and two or more kinds of compounds each represented by the general formula (1) are used as the host material.

In the general formula (1), Z represents a group having an indolocarbazole ring represented by the general formula (2), and a represents an integer of from 1 to 3. The general formula (1) includes in itself the general formula (2), the formula (2a), and the formula (2b), and hence when the term "general formula (1) " is used, the term may be used in such meaning as to include the formulate. Similarly, when the term "general formula (2)" is used, the term may be used in such meaning as to include the formula (2a) and the formula (2b).

In the general formula (2), a ring A represents an aromatic hydrocarbon ring represented by the formula (2a), and a ring B represents a heterocycle represented by the formula (2b). The ring A and the ring B are each fused with an adjacent ring.

In the formulae, Ar¹ and Ar² each independently represent an aromatic hydrocarbon group having 6 to 50 carbon atoms, an aromatic heterocyclic group having 3 to 30 carbon atoms, or a linked aromatic group obtained by linking 2 to 6 of the groups. Ar¹ and Ar² each preferably represent an aromatic hydrocarbon group having 6 to 30 carbon atoms, or an aromatic heterocyclic group having 3 to 20 carbon atoms, and each more preferably represent an aromatic hydrocarbon group having 6 to 20 carbon atoms, an aromatic heterocyclic group having 3 to 15 carbon atoms, or a linked aromatic group obtained by linking 2 to 4 of the groups.

In this description, the term "aromatic group" is interpreted as meaning one or both of an aromatic hydrocarbon group and an aromatic heterocyclic group. The term "linked aromatic group" means a compound in which two or more aromatic hydrocarbon groups, two or more aromatic heterocyclic groups, or two or more groups including both an aromatic hydrocarbon group and an aromatic heterocyclic group are linked to each other through a direct bond.

Specific examples of Ar¹ and Ar² include groups each produced by removing a hydrogen atom from: an aromatic compound, such as benzene, naphthalene, acenaphthene, acenaphthylene, azulene, anthracene, chrysene, pyrene,perylene, phenanthrene, triphenylene, corannulene, coronene, kekulene, ovalene, tetracene, pentacene, fluorene, benzo[a]anthracene, benzo[b]fluoranthene, benzo[a]pyrene, indeno[1,2,3-cd]pyrene, dibenzo[a,h]anthracene, picene, tetraphenylene, anthanthrene, 1,12-benzoperylene, circulene, heptacene, hexacene, pyridine, pyrimidine, triazine, thiophene, isothiazole, thiazole, pyridazine, pyrrole, pyrazole, imidazole, triazole, thiadiazole, pyrazine, furan, isoxazole, oxazole, oxadiazole, quinoline, isoquinoline, quinoxaline, quinazoline, oxadiazole, thiadiazole, benzotriazine, phthalazine, tetrazole, indole, benzofuran, benzothiophene, benzoxazole, benzothiazole, indazole, benzimidazole, benzotriazole, benzisothiazole, benzothiadiazole, purine, pyranone, coumarine, isocoumarine, chromone, dibenzofuran, dibenzothiophene, dibenzoselenophene, or carbazole; or a linked aromatic compound in which a plurality of the aromatic compounds are linked to each other.

Those aromatic hydrocarbon groups or aromatic heterocyclic groups may each have a substituent. When any such group has a substituent, the substituent is a cyano group, an aliphatic hydrocarbon group having 1 to 10 carbon atoms, a diarylamino group having 12 to 44 carbon atoms, or a diarylboryl group having 12 to 44 carbon atoms. The number of the substituents is desirably from 0 to 5, preferably from 0 to 2, When any such aromatic hydrocarbon group or aromatic heterocyclic group has a substituent, the number of carbon atoms of the substituent is not included in the calculation of the number of carbon atoms. However, the total number of carbon atoms including the number of carbon atoms of the substituent preferably satisfies the above-mentioned range.

Specific examples of the substituent include cyano, methyl, ethyl, propyl, butyl, pentyl, hexyl, heptyl, octyl, nonyl, decyl, diphenylamino, naphthylphenylamino, dinaphthylamino, dianthranylamino, diphenanthrenylamino, dipyrenylamino, diphenylboryl, dinaphthylboryl, and dianthranylboryl. Of those, cyano, methyl, ethyl, propyl, butyl, pentyl, hexyl, heptyl, octyl, diphenylamino, naphthylphenylamino, or dinaphthylamino is preferred.

R represents a cyano group, an aliphatic hydrocarbon group having 1 to 10 carbon atoms, a diarylamino group having 12 to 44 carbon atoms, a diarylboryl group having 12 to 44 carbon atoms, an aromatic hydrocarbon group having 6 to 18 carbon atoms, or an aromatic heterocyclic group having 3 to 18 carbon atoms, preferably a cyano group, an aliphatic hydrocarbon group having 1 to 8 carbon atoms , a diarylamino group having 12 to 2 0 carbon atoms, a diarylboryl group having 12 to 20 carbon atoms, an aromatic hydrocarbon group having 6 to 10 carbon atoms, or an aromatic heterocyclic group having 3 to 15 carbon atoms, more preferably an aromatic hydrocarbon group having 6 to 10 carbon atoms or an aromatic heterocyclic group having 3 to 15 carbon atoms.

When R represents an aliphatic hydrocarbon group having 1 to 10 carbon atoms, a diarylamino group having 12 to 44 carbon atoms, or a diarylboryl group having 12 to 44 carbon atoms, specific examples thereof include methyl, ethyl, propyl, butyl, pentyl, hexyl, heptyl, octyl, nonyl, decyl, diphenylamino, naphthylphenylamino, dinaphthylamino, dianthranylamino, diphenanthrenylamino, dipyrenylamino, diphenylboryl, dinaphthylboryl, and dianthranylboryl. Of those, methyl, ethyl, propyl, butyl, pentyl, hexyl, heptyl, octyl, diphenylamino, naphthylphenylamino, or dinaphthylamino is preferred.

When R represents an aromatic hydrocarbon group having 6 to 18 carbon atoms or an aromatic heterocyclic group having 3 to 18 carbon atoms, specific examples thereof include aromatic groups each produced by removing a H from benzene, naphthalene, anthracene, pyrene, phenanthrene, triphenylene, fluorene, pyridine, pyrimidine, triazine, thiophene, isothiazole, thiazole, pyridazine, pyrrole, pyrazole, imidazole, triazole, thiadiazole, pyrazine, furan, isoxazole, oxazole, oxadiazole, quinoline, isoquinoline, quinoxaline, quinazoline, oxadiazole, thiadiazole, benzotriazine, phthalazine, tetrazole, indole, benzofuran, benzothiophene, benzoxazole, benzothiazole, indazole, benzimidazole, benzotriazole, benzisothiazole, benzothiadiazole, purine, pyranone, coumarine, isocoumarine, chromone, dibenzofuran, dibenzothiophene, dibenzoselenophene, carbazole, or a linked aromatic compound in which a plurality of these compounds are linked to each other. Here, in the case of an aromatic group produced from a linked aromatic compound, when a ring to be bonded to the indolocarbazole ring of the general formula (2) is an aromatic hydrocarbon ring, the group is defined as an aromatic hydrocarbon group, and when the ring is an aromatic heterocycle, the group is defined as an aromatic heterocyclic group.

L¹ and L² each independently represent an aromatic hydrocarbon group having 6 to 50 carbon atoms, or an aromatic heterocyclic group having 3 to 30 carbon atoms. L¹ and L² each preferably represent an aromatic hydrocarbon group having 6 to 30 carbon atoms, or an aromatic heterocyclic group having 3 to 20 carbon atoms. L¹ and L² each more preferably represent an aromatic hydrocarbon group having 6 to 20 carbon atoms, or an aromatic heterocyclic group having 3 to 15 carbon atoms. Two or more kinds of compounds each represented by the general formula (1) are used, and one of L¹ and L² of at least one kind of the compounds preferably represents a nitrogen-containing aromatic heterocyclic group having 3 to 30 carbon atoms. However, it is not desirable that both of L¹ and L² each represent a nitrogen-containing aromatic heterocyclic group.

Specific examples of L¹ and L² include groups each produced by removing a+b or f+1 H's from benzene, naphthalene, acenaphthene, acenaphthylene, azulene, anthracene, chrysene, pyrene, perylene, phenanthrene, triphenylene, corannulene, coronene, kekulene, ovalene, tetracene, pentacene, fluorene, benzo[a]anthracene, benzo[b]fluoranthene, benzo[a]pyrene, indeno[1,2,3-cd]pyrene, diberlzo[a,h]anthracene, picene, tetraphenylene, anthanthrene, 1, 12-benzoperylene, circulene, heptacene, hexacene, pyridine, pyrimidine, triazine, thiophene, isothiazole, thiazole, pyridazine, pyrrole, pyrazole, imidazole, triazole, thiadiazole, pyrazine, furan, isoxazole, oxazole, oxadiazole, quinoline, isoquinoline, quinoxaline, quinazoline, oxadiazole, thiadiazole, benzotriazine, phthalazine, tetrazole, indole, benzofuran, benzothiophene, benzoxazole, benzothiazole, indazole, benzimidazole, benzotriazole, benzisothiazole, benzothiadiazole, purine, pyranone, coumarine, isocoumarine, chromone, dibenzofuran, dibenzothiophene, dibenzoselenophene, or carbazole.

a represents an integer of from 1 to 3, preferably an integer of 1 or 2. b represents an integer of from 0 to 3, preferably an integer of 1 or 2. f represents an integer of from 0 to 3 , preferably an integer of 0 or 1. c and d each independently represent an integer of from 0 to 4, preferably an integer of from 0 to 2. e represents an integer of from 0 to 2, preferably an integer of 0 or 1. It is more preferred that c to e each independently represent 0 or 1, and c+e+d be from 0 to 2.

When Ar¹, Ar², R¹, or R² represents a linked aromatic group in which a plurality of aromatic hydrocarbon groups or aromatic heterocyclic groups are linked to each other, such linking systems as shown below are given.

-Ar-Ar-Ar (5)

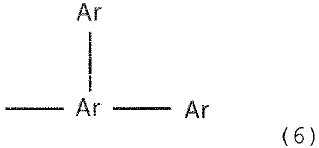

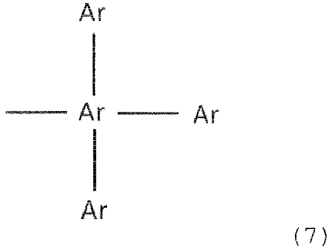

-Ar-Ar- (8)

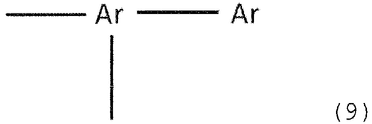

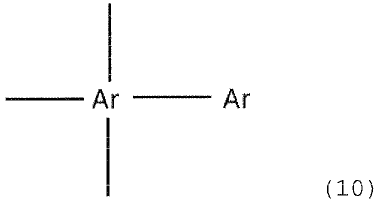

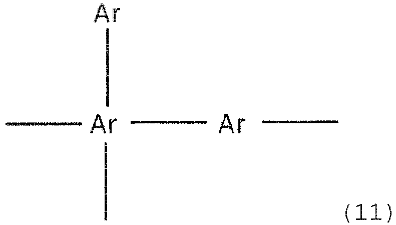

In the formulae, Ar' s each independently represent an aromatic hydrocarbon group or an aromatic heterocyclic group, and may each represent a fused ring. Each of Ar' s may have a substituent, but does not have an aromatic group as a substituent.

Specific examples of the group in which a plurality of aromatic hydrocarbon groups or aromatic heterocyclic groups are linked to each other include biphenyl, terphenyl, quaterphenyl, bipyridine, bitriazine, terpyridine, binaphthalene, phenylpyridine, diphenylpyridine, phenylpyrimidine, diphenylpyrimidine, diphenyltriazine, phenylnaphthalene, diphenylnaphthalene, carbazolylbenzene, biscarbazole, biscarbazolylbenzene, biscarbazolyltriazine, dibenzofuranylbenzene, bisdibenzofuranylbenzene, dibenzothienylbenzene, and bisdibenzothienylbenzene.

When L¹, L², Ar¹, Ar², and R's each represent an aromatic heterocyclic group, the group is not an aromatic heterocyclic group having an indolocarbazole ring represented by the general formula (2). Here, the term "indolocarbazole ring" refers to a pentacyclic fused ring in the general formula (2).

Preferred specific examples of the compounds each represented by the general formula (1) are shown below, but the compounds are not limited thereto.

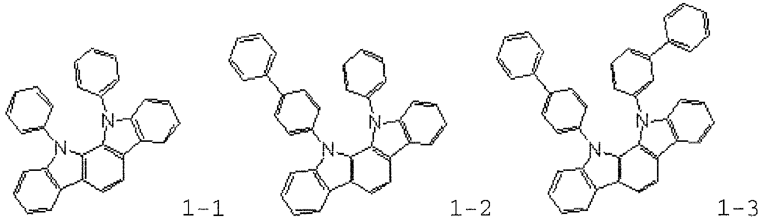

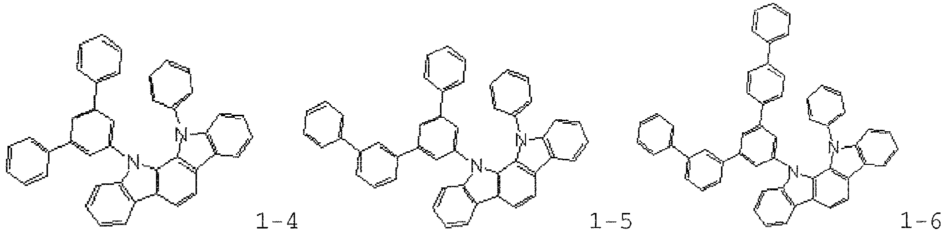

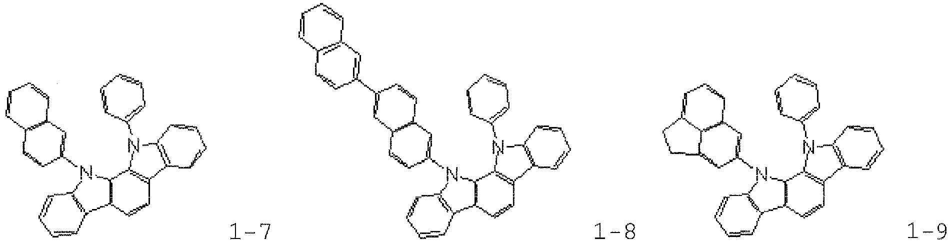

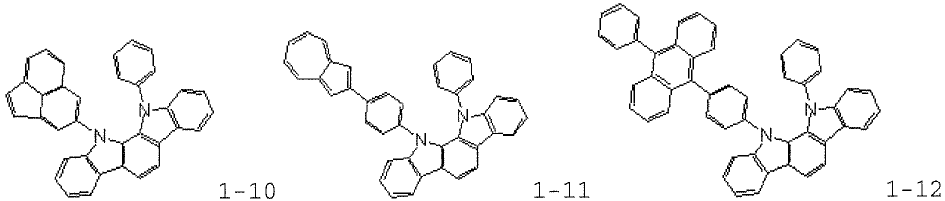

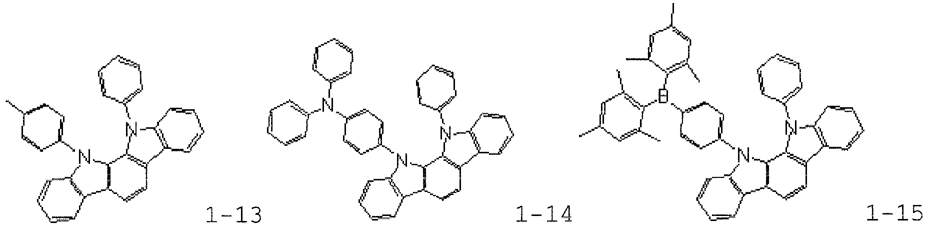

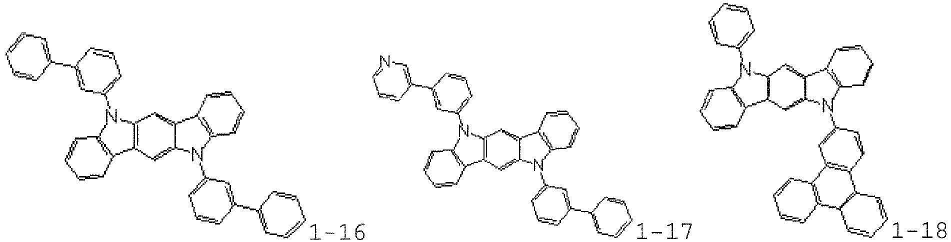

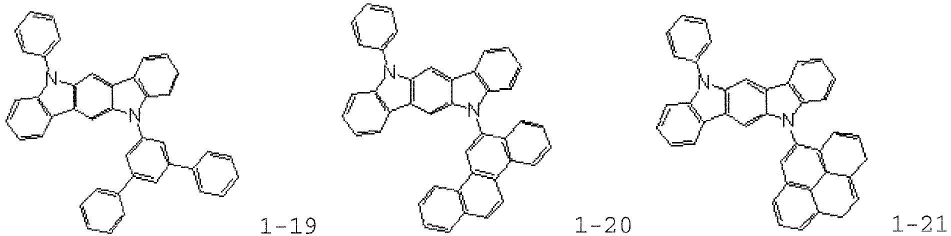

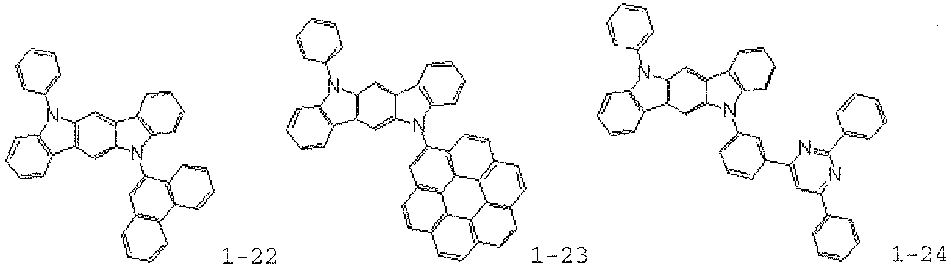

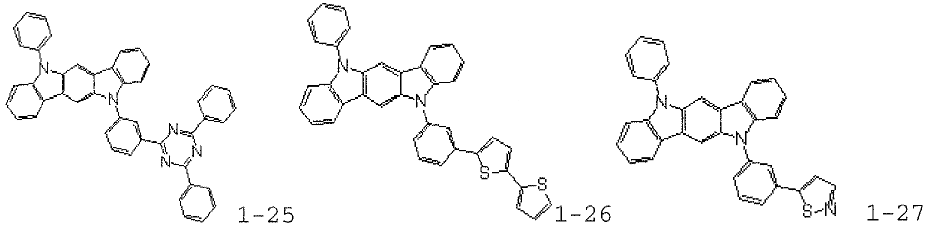

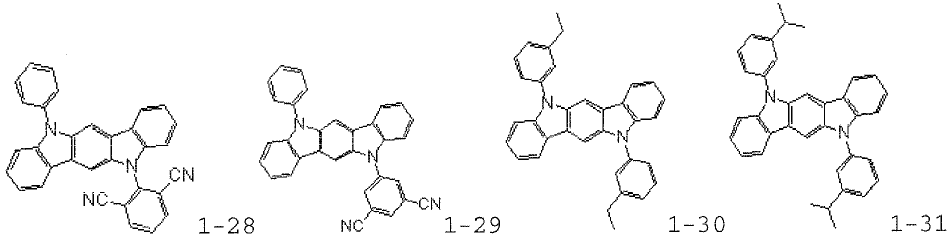

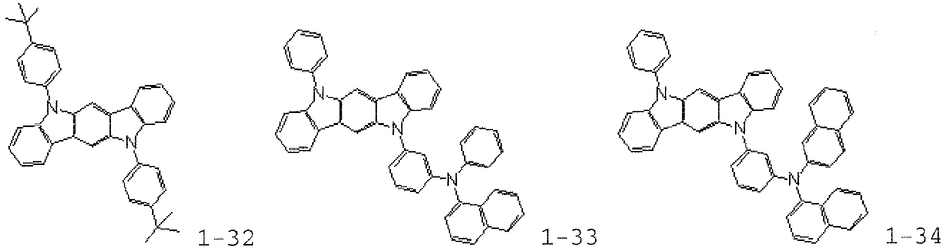

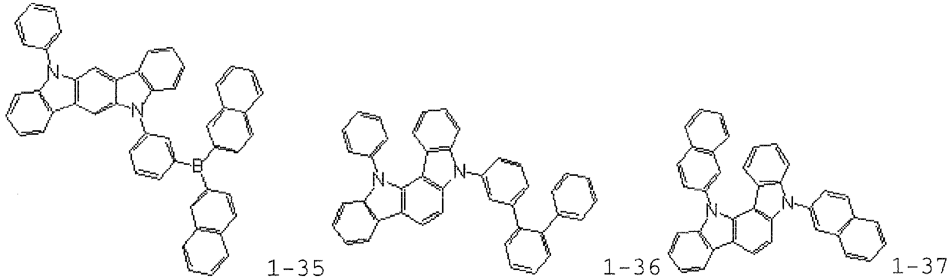

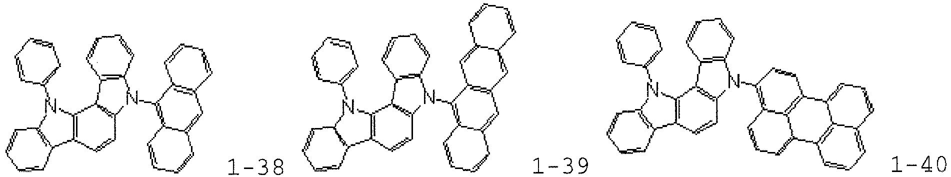

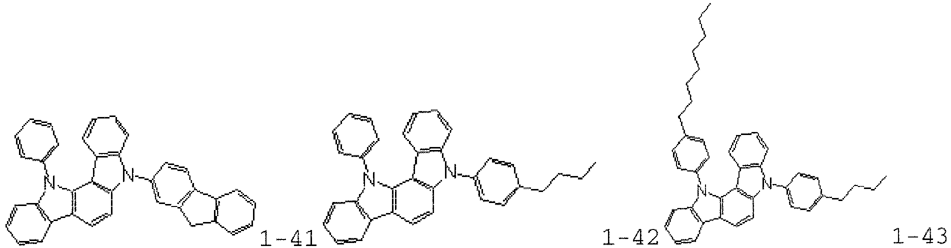

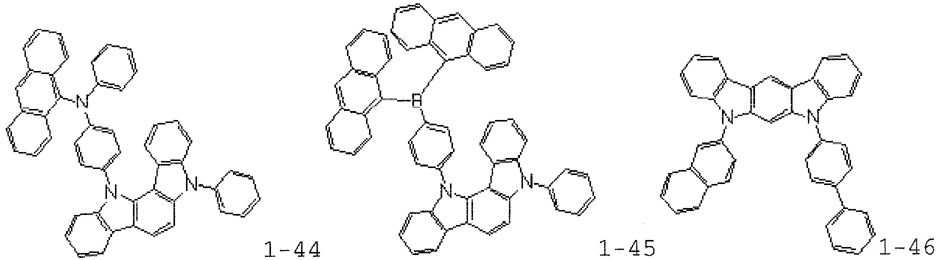

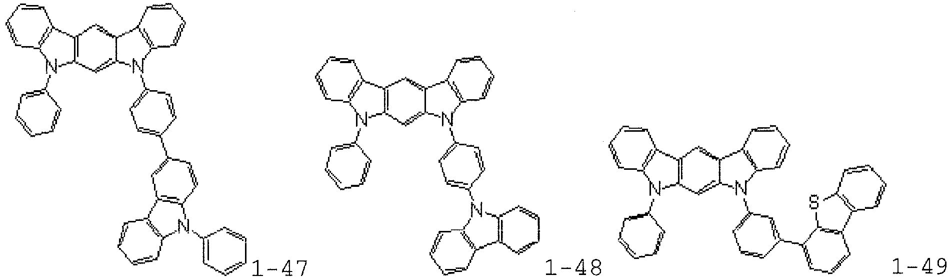

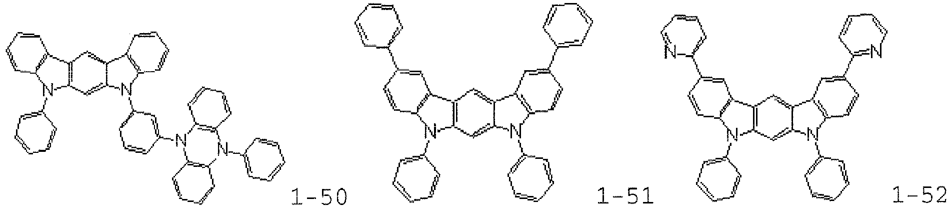

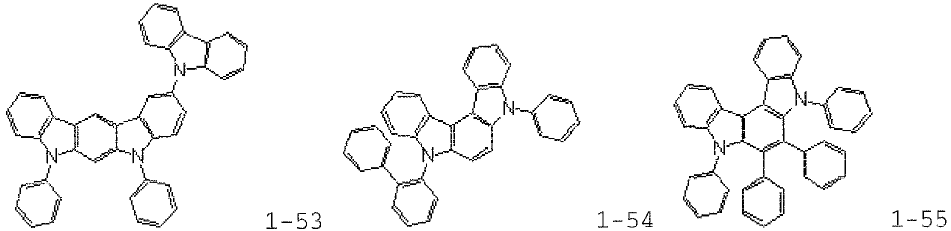

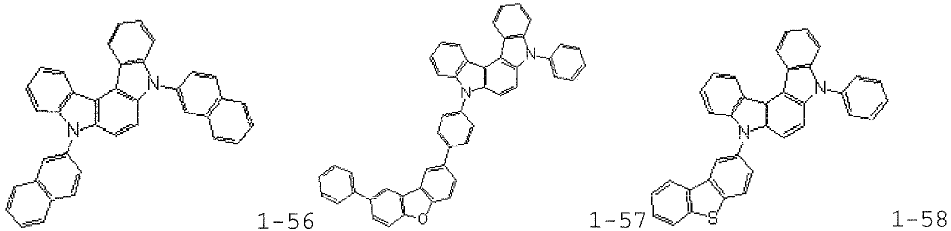

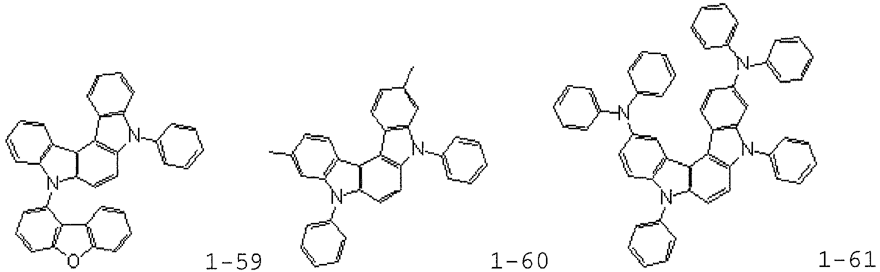

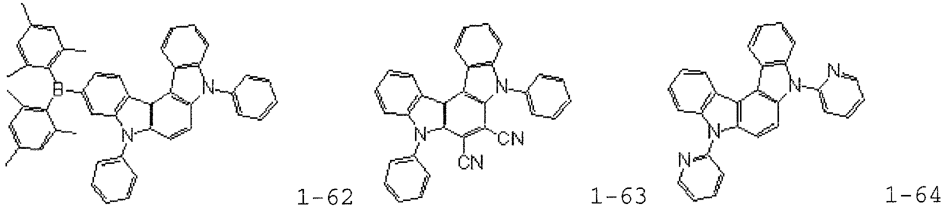

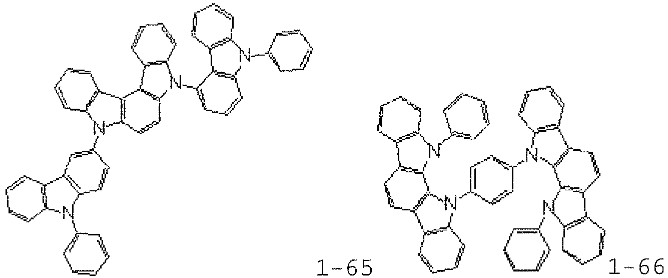

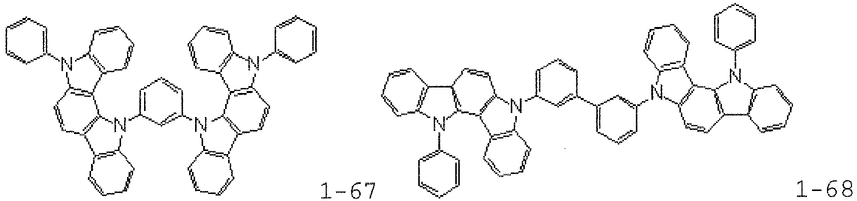

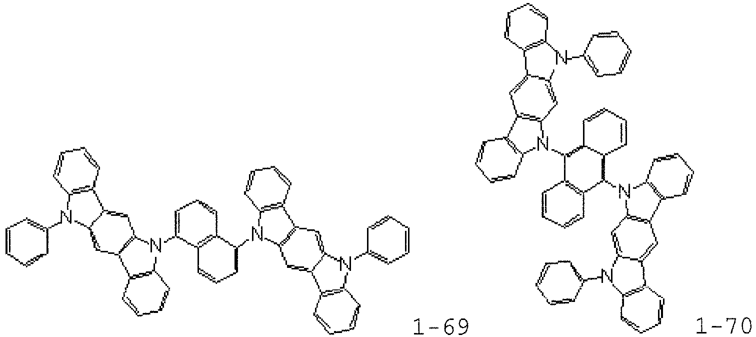

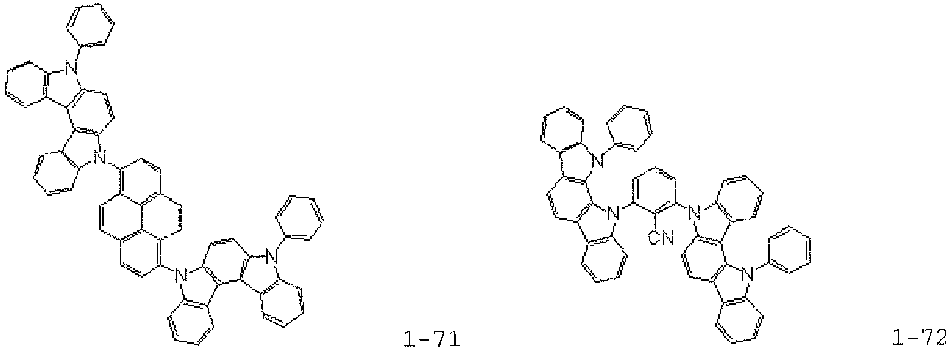

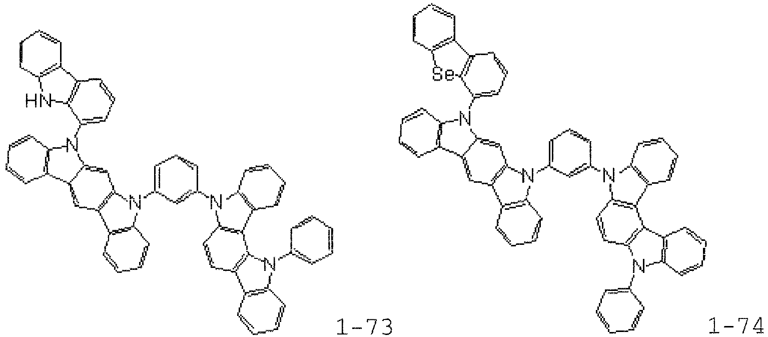

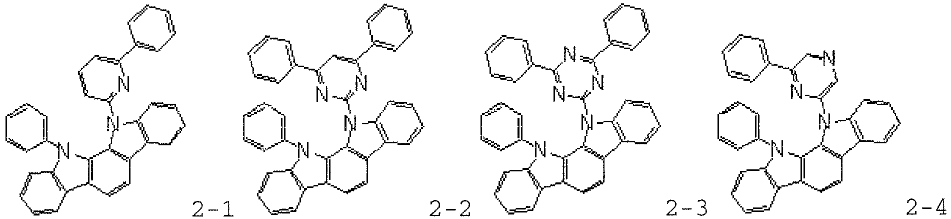

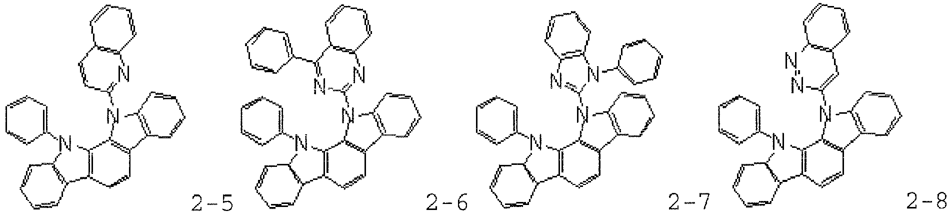

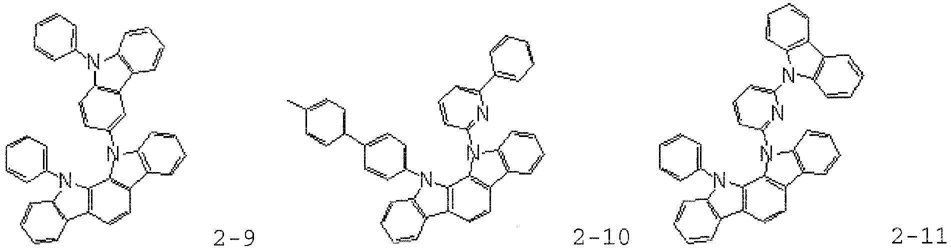

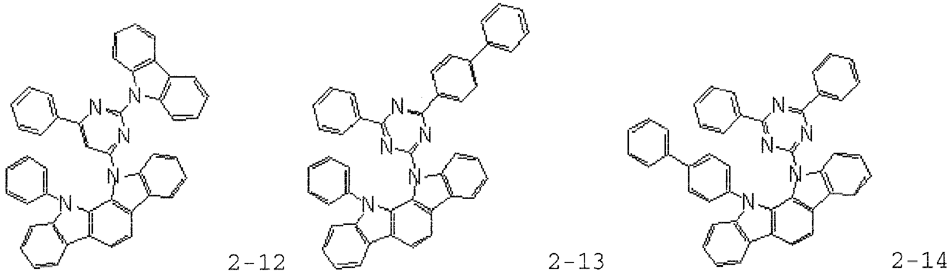

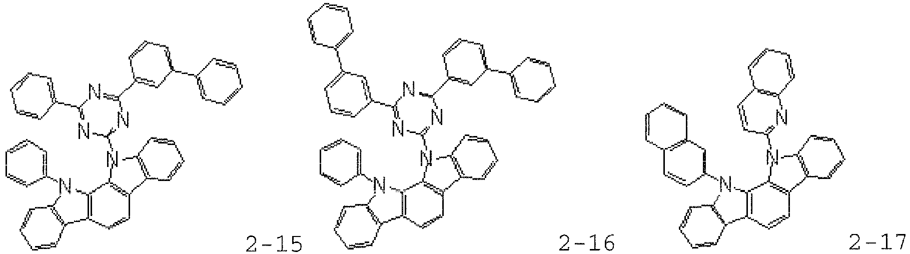

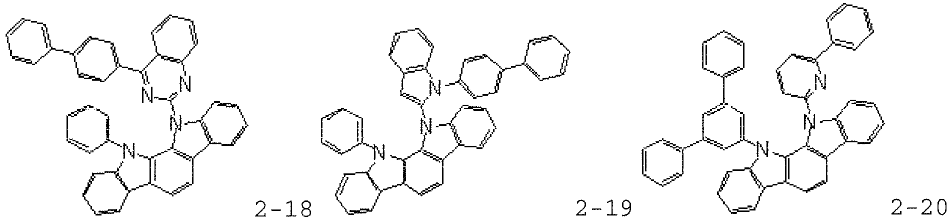

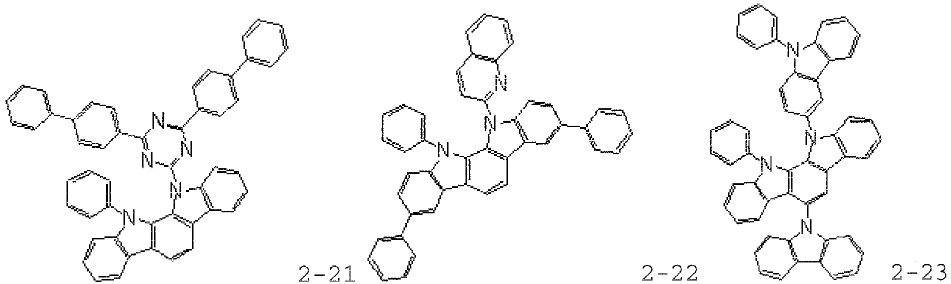

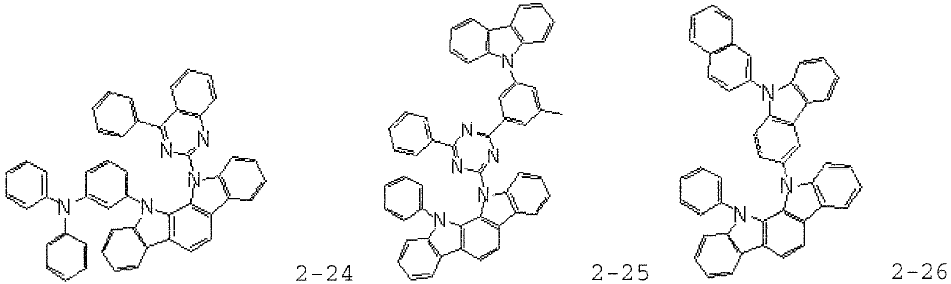

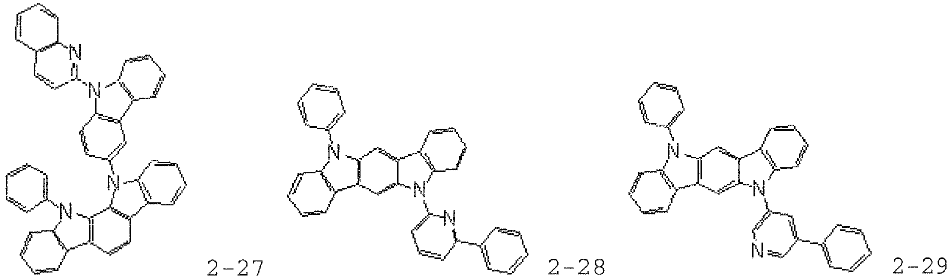

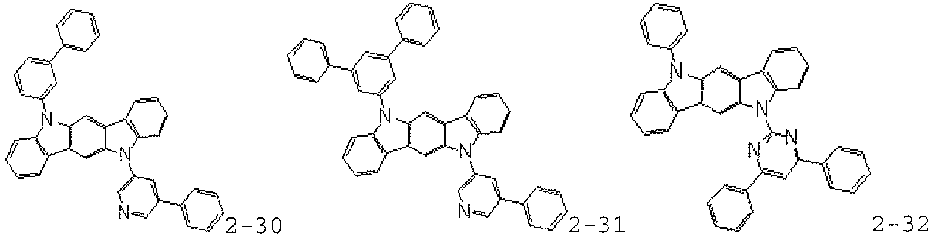

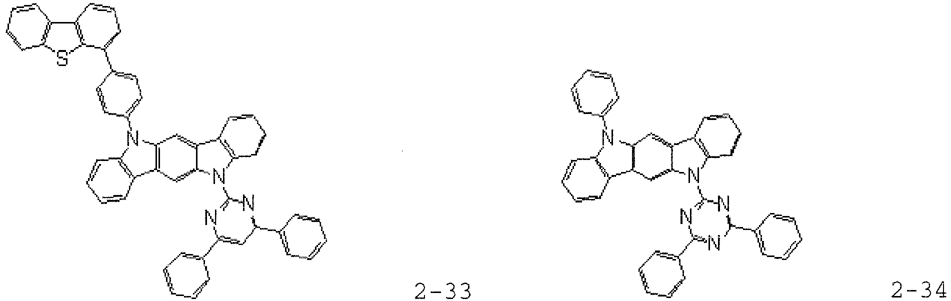

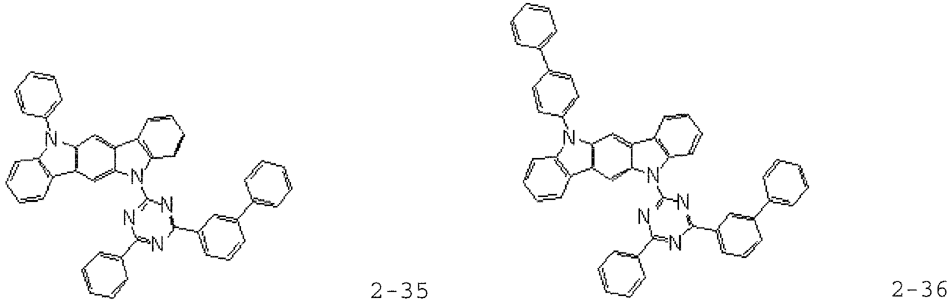

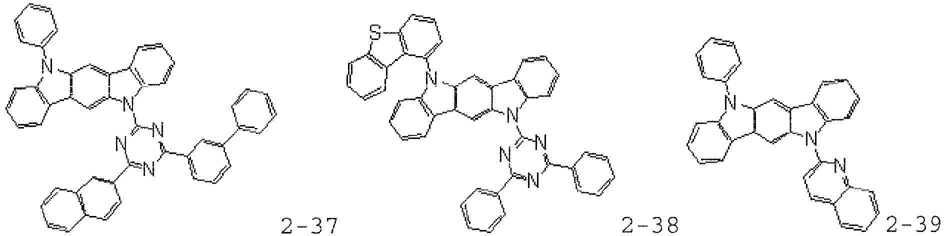

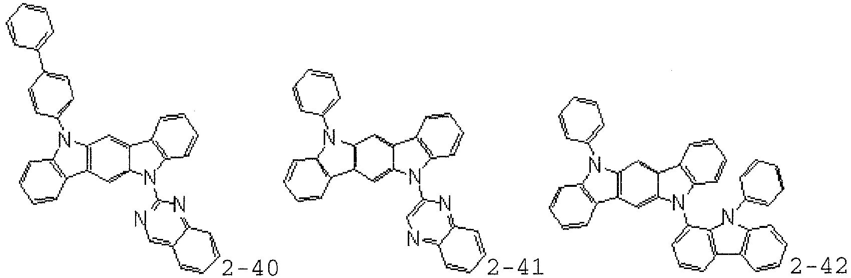

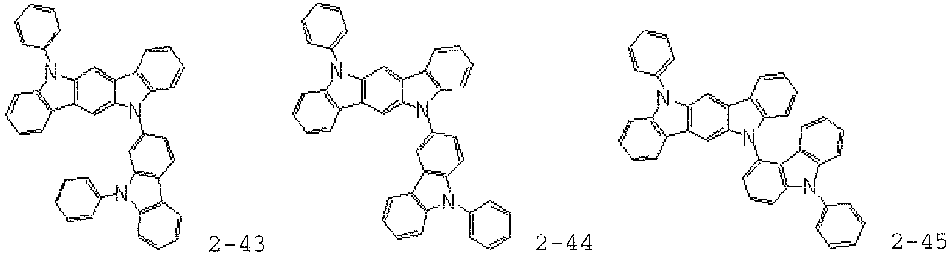

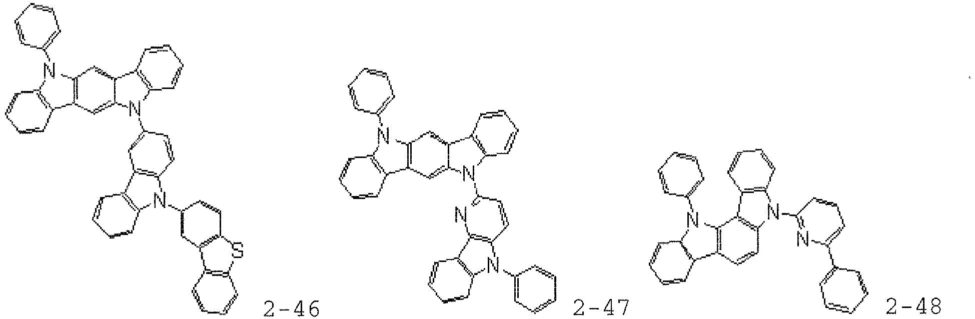

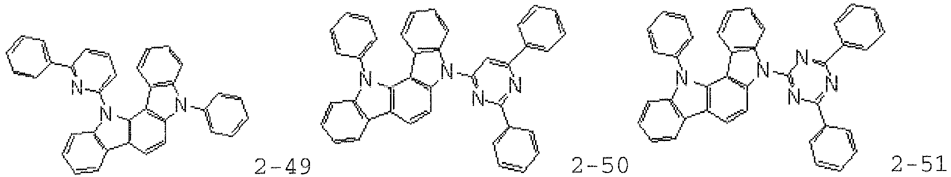

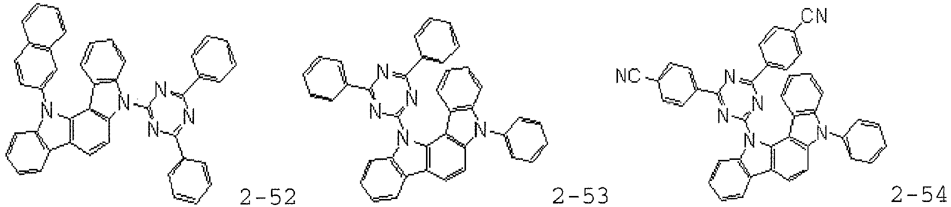

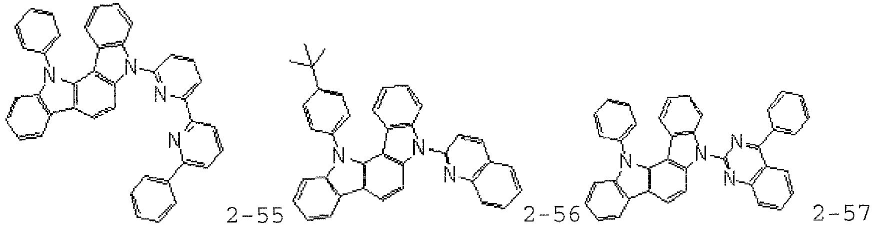

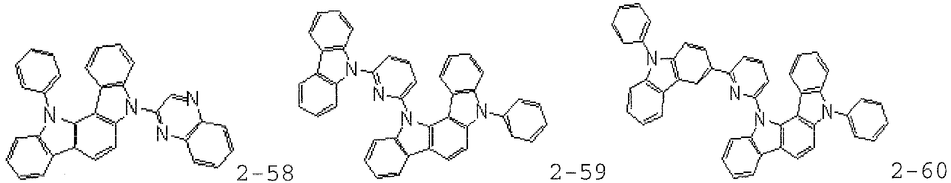

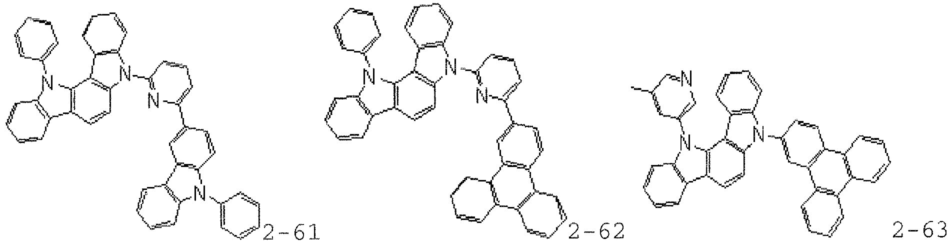

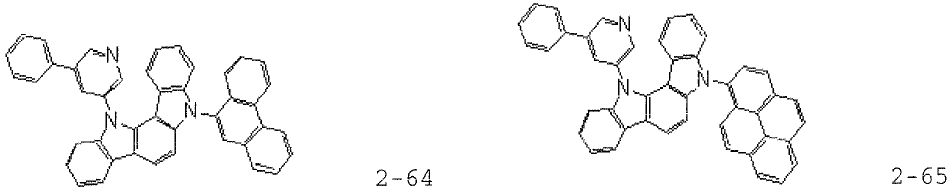

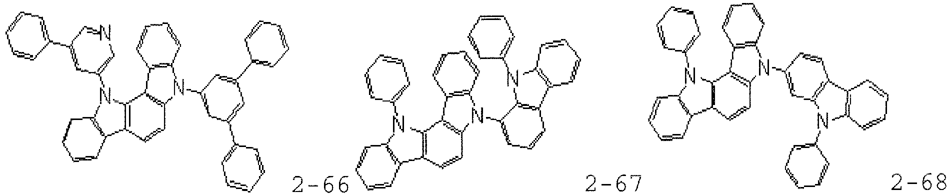

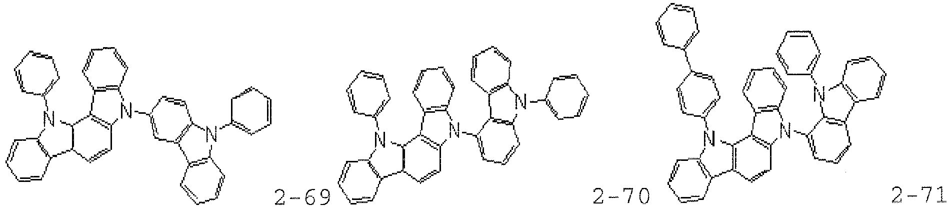

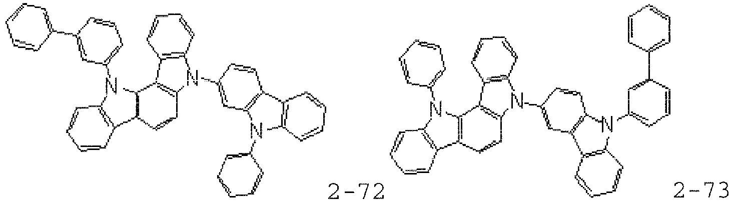

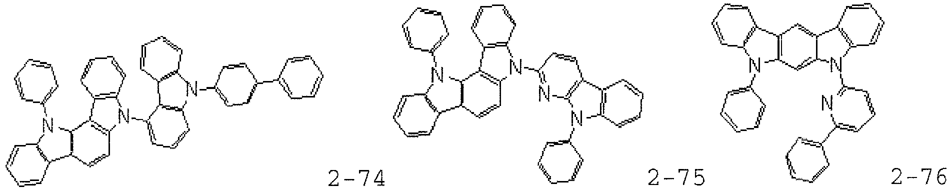

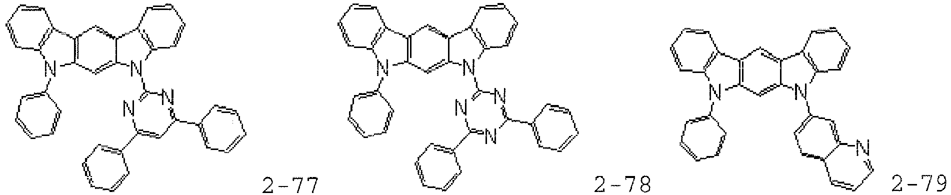

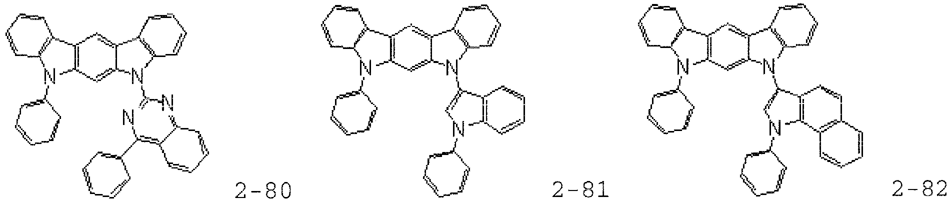

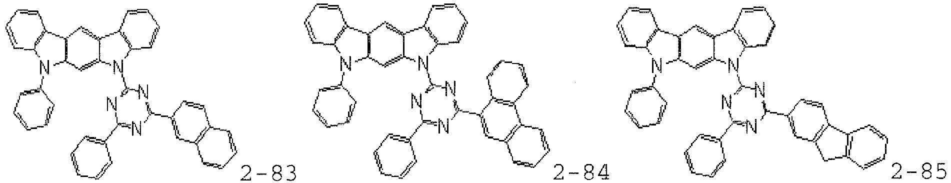

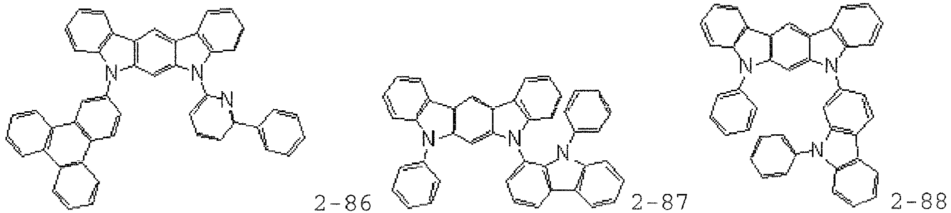

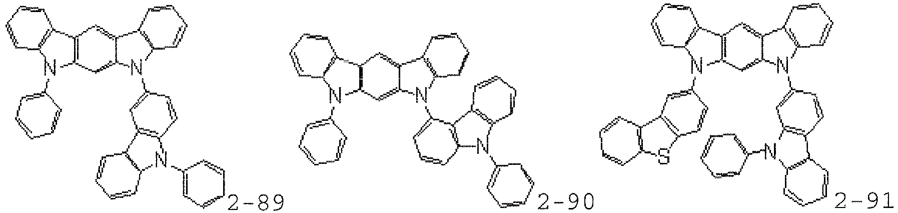

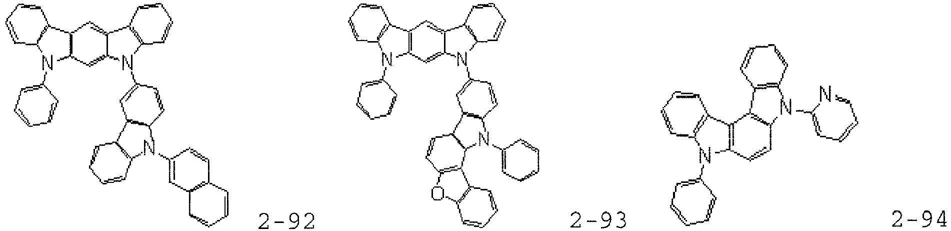

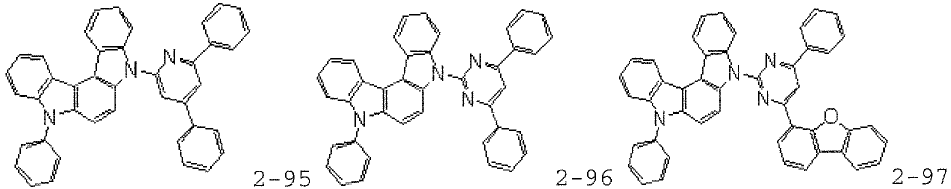

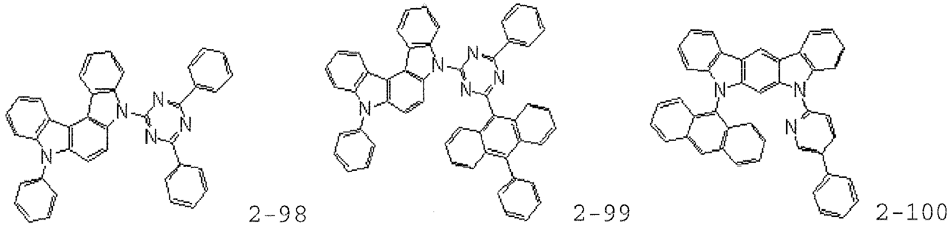

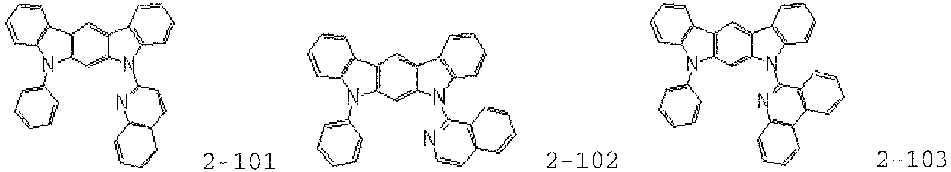

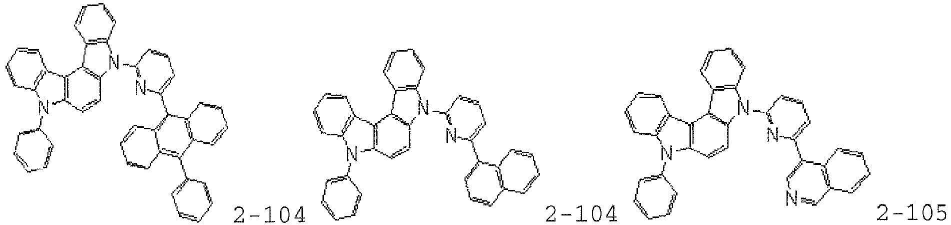

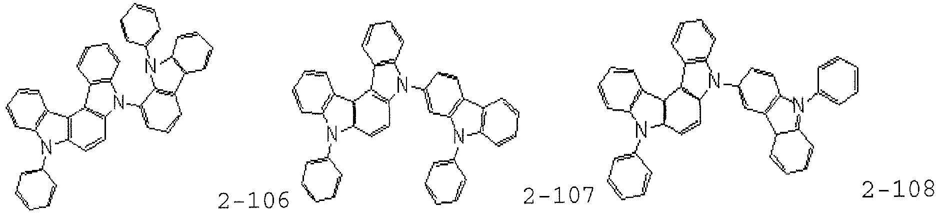

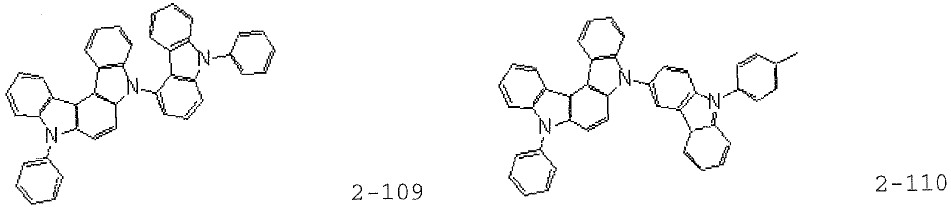

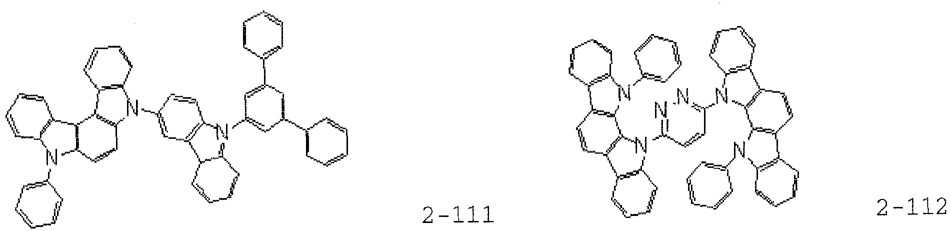

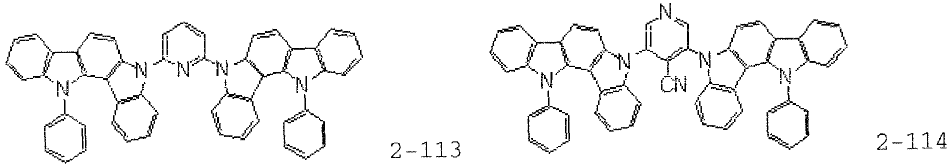

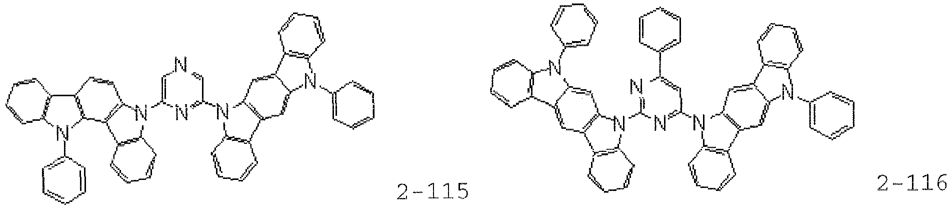

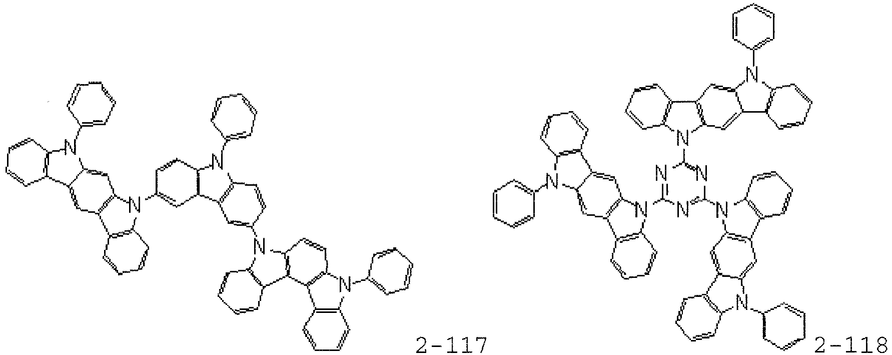

An excellent organic EL device can be provided by using, as a host material for a light-emitting layer, a material obtained by preliminarily mixing two or more kinds of compounds selected from the compounds each represented by the general formula (1). Although a mixing ratio (weight ratio) between the two kinds of compounds to be preliminarily mixed is not particularly limited, the ratio preferably falls within the range of from 95:5 to 5:95, and more preferably falls within the range of from 90:10 to 10:90.

Next, the structure of the organic EL device of the present invention is described with reference to the drawings. However, the structure of the organic EL device of the present invention is not limited to one illustrated in the drawings.

FIG. 1 is a sectional view for illustrating a structure example of a general organic EL device used in the present invention. Reference numeral 1 represents a substrate, reference numeral 2 represents an anode, reference numeral 3 represents a hole-injecting layer, reference numeral 4 represents a hole-transporting layer, reference numeral 5 represents a light-emitting layer, reference numeral 6 represents an electron-transporting layer, and reference numeral 7 represents a cathode. The organic EL device of the present invention may include an exciton-blocking layer adjacent to the light-emitting layer, or may include an electron-blocking layer between the light-emitting layer and the hole-inj ecting layer. The exciton-blocking layer may be inserted on any of the cathode side and the cathode side of the light-emitting layer, and may also be inserted simultaneously on both sides. The organic EL device of the present invention includes the anode, the light-emitting layer, and the cathode as its essential layers. The organic EL device of the present invention preferably includes a hole-injecting/transporting layer and an electron-injecting/transporting layer in addition to the essential layers, and more preferably includes a hole-blocking layer between the light-emitting layer and the electron-injecting/transporting layer. The hole-injecting/transporting layer means any one or both of the hole-injecting layer and the hole-transporting layer, and the electron-injecting/transporting layer means any one or both of an electron-injecting layer and the electron-transporting layer.

It is possible to adopt a reverse structure as compared to FIG. 1, that is, the reverse structure being formed by laminating the layers on the substrate 1 in the order of the cathode 7, the electron-transporting layer 6, the light-emitting layer 5, the hole-transporting layer 4, and the anode 2. In this case as well, some layers may be added or eliminated if necessary.

### -Substrate-

The organic EL device of the present invention is preferably supported by a substrate. The substrate is not particularly limited, and any substrate that has been conventionally used for an organic EL device may be used. For example, a substrate made of glass, a transparent plastic, quartz, or the like may be used.

### -Anode-

Preferably used as the anode in the organic EL device is an anode formed by using, as an electrode substance, any of a metal, an alloy, an electrically conductive compound, and a mixture thereof, all of which have a large work function (4 eV or more). Specific examples of such electrode substance include metals, such as Au, and conductive transparent materials, such as CuI, indium tin oxide (ITO), SnO₂, and ZnO. Further, it may be possible to use a material such as IDIXO (In₂O₃-ZnO), which may be used for manufacturing an amorphous, transparent conductive film. In order to produce the anode, it may be possible to form any of those electrode substances into a thin film by using a method such as vapor deposition or sputtering and form a pattern having a desired shape thereon by photolithography. Alternatively, in the case of not requiring high pattern accuracy (about 100 µm or more), a pattern may be formed via a mask having a desired shape when any of the above-mentioned electrode substances is subj ected to vapor deposition or sputtering. Alternatively, when a coatable substance, such as an organic conductive compound, is used, it is also possible to use a wet film-forming method, such as a printing method or a coating method. When luminescence is taken out from the anode, the transmittance of the anode is desirably controlled to more than 10%. Further, the sheet resistance as the anode is preferably several hundred Ω/□ or less. Further, the thickness of the film is, depending on its material, selected from usually the range of from 10 nm to 1,000 nm, preferably the range of from 10 nm to 200 nm.

### -Cathode-

On the other hand, used as the cathode is a cathode formed by using, as an electrode substance, any of a metal (referred to as electron-injecting metal), an alloy, an electrically conductive compound, and a mixture thereof, all of which have a small work function (4 eV or less). Specific examples of such electrode substance include sodium, a sodium-potassium alloy, magnesium, lithium, a magnesium/copper mixture, a magnesium/silver mixture, a magnesium/aluminum mixture, a magnesium/indium mixture, an aluminum/aluminum oxide (Al₂O₃) mixture, indium, a lithium/aluminum mixture, and a rare earth metal. Of those, for example, a mixture of an electron-injecting metal and a second metal as a stable metal having a larger work function value than that of the former metal, such as a magnesium/silver mixture, a magnesium/aluminum mixture, a magnesium/indium mixture, an aluminum/aluminum oxide (Al₂O₃) mixture, or a lithium/aluminum mixture, or aluminum is suitable from the viewpoints of an electron-inj ecting property and durability against oxidation or the like . The cathode may be produced by forming any of those electrode substances into a thin film by using a method such as vapor deposition or sputtering. Further, the sheet resistance as the cathode is preferably several hundred Ω/□ or less, and the thickness of the film is selected from usually the range of from 10 nm to 5 µm, preferably the range of from 50 nm to 200 nm. Any one of the anode and cathode of the organic EL device is preferably transparent or semi-transparent because emitted light is transmitted therethrough and the light emission luminance improves.

Further, after any of the above-mentioned metals is formed into a film having a thickness of from 1 nm to 20 nm as a cathode, any of the conductive transparent materials mentioned in the description of the anode is formed into a film on the cathode, thereby being able to produce a transparent or semi-transparent cathode. Then, by applying this, it is possible to produce a device in which both the anode and cathode have transparency.

### -Light-emitting Layer-

The light-emitting layer is a layer that emits light after the production of an exciton by the recombination of a hole injected from the anode and an electron injected from the cathode, and the light-emitting layer contains an organic light-emitting dopant material and a host material.

A material obtained by preliminarily mixing two or more kinds of compounds each represented by the general formula (1) at an arbitrary ratio before deposition is used as the host material in the light-emitting layer. That is, two or more kinds of compounds, preferably two or three kinds of compounds selected from the compounds each represented by the general formula (1) are preliminarily mixed.

A difference in evaporation temperature between the two or more kinds of compounds is desirably as small as possible in order that an organic EL device having satisfactory characteristics may be produced with good reproducibility. In this description, the temperature at which the weight of a compound reduces by 50% in TG-DTAmeasurement in a vacuum (50 Pa) is defined as an evaporation temperature.

The difference in evaporation temperature between the two or more kinds of compounds is preferably 20°C or less, more preferably 10°C or less. When the host material is formed of three or more kinds of compounds, the evaporation temperature of a compound showing an intermediate evaporation temperature is used as a reference, and a difference between the reference and the evaporation temperature of any other compound is preferably 20°C or less. A known method, such as pulverization mixing, may be adopted as a method for the preliminary mixing, but it is desirable that the compounds be mixed as uniformly as possible.

In addition, one or more kinds of other host materials, such as known host materials, may be used as host materials in the light-emitting layer in addition to the two or more kinds of compounds each represented by the general formula (1), but the usage amount of the other host materials is desirably set to 50 wt% or less, preferably 25 wt% or less with respect to the total of the host materials.

The other host materials are not particularly limited, and examples thereof include: compounds each having a fused aromatic ring, such as naphthalene, anthracene, phenanthrene, triphenylene, fluorene, and indene, and derivatives thereof; aromatic amine derivatives, such as N,N'-dinaphthyl-N,N'-diphenyl-4,4'-diphenyl-1-diamine; metal complexes, such as tris(8-quinolinato)aluminum(III); and derivatives of a compound having a heterocycle, such as a dibenzofuran derivative, a dibenzothiophene derivative, a carbazole derivative, a dicarbazole derivative, a pyridine derivative, a pyrimidine derivative, and a triazine derivative.

When a phosphorescent light-emitting dopant is used as the organic light-emitting dopant material, the phosphorescent light-emitting dopant is preferably a phosphorescent light-remitting dopant containing an organometallic complex containing at least one metal selected from ruthenium, rhodium, palladium, silver, rhenium, osmium, iridium, platinum, and gold. Specifically, an iridium complex disclosed in J. Am. Chem. Soc. 2001, 123, 4304 or JP 2013-53051 A is suitably used, but the organometallic complex is not limited thereto.

Only one kind of phosphorescent light-emitting dopant material may be incorporated into the light-emitting layer, or two or more kinds of phosphorescent light-emitting dopant materials may be incorporated into the layer. When the layer contains two or more kinds of phosphorescent light-emitting dopant materials, the total weight of the phosphorescent light-emitting dopant materials is preferably 30% or less, more preferably 20% or less with respect to the host material.

The phosphorescent light-emitting dopant material is not particularly limited, but specific examples thereof include the following materials.

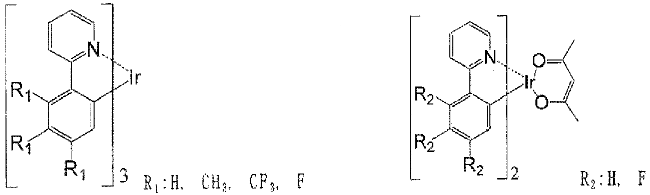

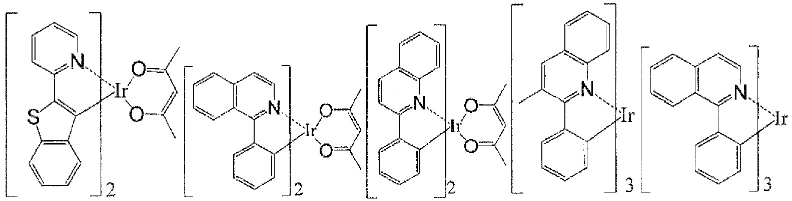

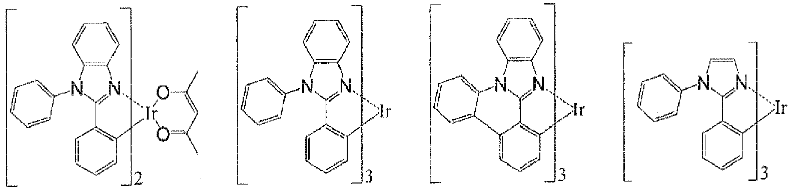

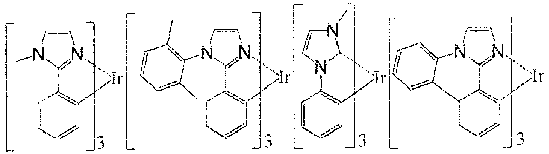

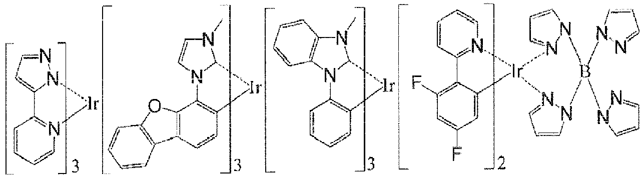

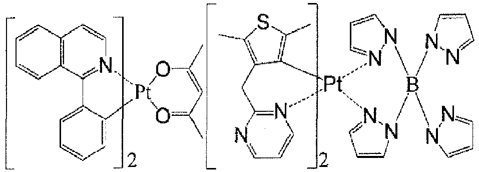

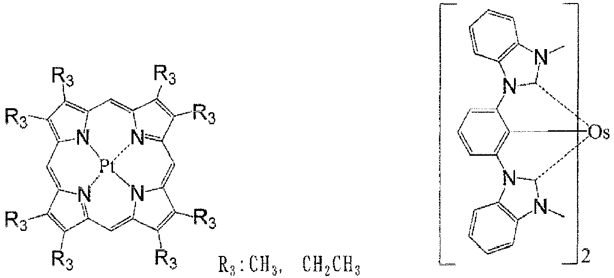

When a fluorescent light-emitting dopant is used as the light-emitting dopant material, the fluorescent light-remitting dopant is not particularly limited, and examples thereof include a benzoxazole derivative, a benzothiazole derivative, a benzimidazole derivative, a styrylbenzene derivative, a polyphenyl derivative, a diphenylbutadiene derivative, a tetraphenylbutadiene derivative, a naphthalimide derivative, a coumarine derivative, a fused aromatic compound, a perinone derivative, an oxadiazole derivative, an oxazine derivative, an aldazine derivative, a pyrrolidine derivative, a cyclopentadiene derivative, a bisstyrylanthracene derivative, a quinacridone derivative, a pyrrolopyridine derivative, a thiadiazolopyridine derivative, a styrylamine derivative, a diketopyrrolopyrrole derivative, an aromatic dimethylidyne compound, various metal complexes typified by a metal complex of an 8-quinolinol derivative, and a metal complex, rare earth complex, or transition metal complex of a pyrromethene derivative, polymer compounds, such as polythiophene, polyphenylene, and polyphenylene vinylene, and an organic silane derivative. Of those, for example, the following compound is preferred: a fused aromatic derivative, a styryl derivative, a diketopyrrolopyrrole derivative, an oxazine derivative, a pyrromethene metal complex, a transition metal complex, or a lanthanoid complex. For example, the following compound is more preferred: naphthacene, pyrene, chrysene, triphenylene, benzo[c]phenanthrene, benzo[a]anthracene, pentacene, perylene, fluoranthene, acenaphthofluoranthene, dibenzo[a,j]anthracene, dibenzo[a,h]anthracene, benzo[a]naphthacene, hexacene, naphtho[2,1-f]isoquinoline, α-naphthaphenanthridine, phenanthroxazole, quinolino[6,5-f]quinoline, or benzothiophanthrene.

Those compounds may each have an alkyl group, an aryl group, an aromatic heterocyclic group, or a diarylamino group as a substituent.

Only one kind of fluorescent light-emitting dopant material may be incorporated into the light-emitting layer, or two or more kinds of fluorescent light-emitting dopant materials may be incorporated into the layer. When the layer contains two or more kinds of fluorescent light-emitting dopant materials, the total weight of the fluorescent light-emitting dopant materials is preferably 20% or less, more preferably 10% or less with respect to the host material.

When a thermally activated delayed fluorescent light-emitting dopant is used as the organic light-emitting dopant material, the thermally activated delayed fluorescent light-emitting dopant is not particularly limited, and examples thereof include: metal complexes, such as a tin complex and a copper complex; indolocarbazole derivatives disclosed in WO 2011/070963 A; cyanobenzene derivatives and carbazole derivatives disclosed in Nature 2012, 492, 234; and phenazine derivatives, oxadiazole derivatives, triazole derivatives, sulfone derivatives, phenoxazine derivatives, and acridine derivatives disclosed in Nature Photonics 2014, 8, 326.

The thermally activated delayed fluorescent light-emitting dopant material is not particularly limited, but specific examples thereof include the following materials.

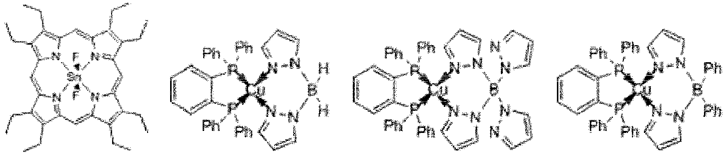

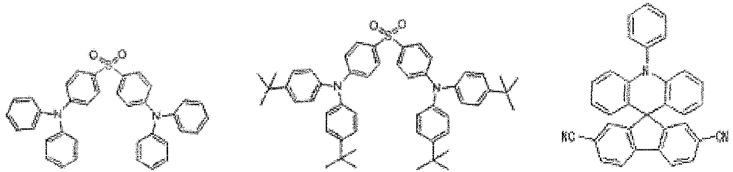

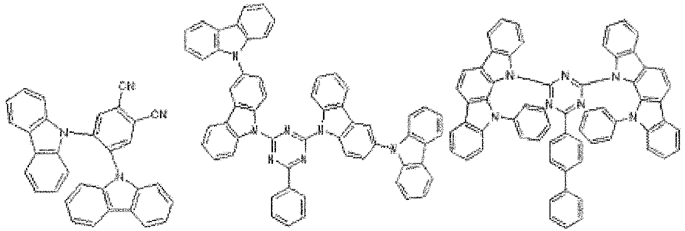

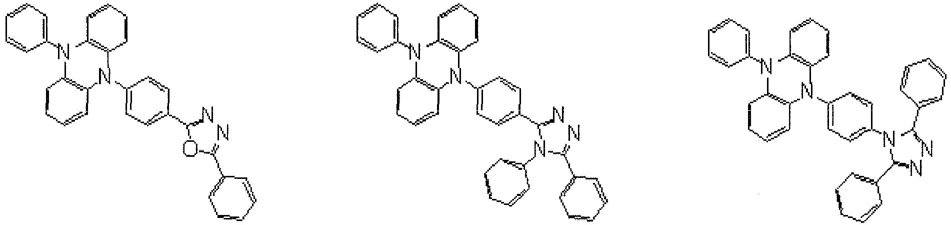

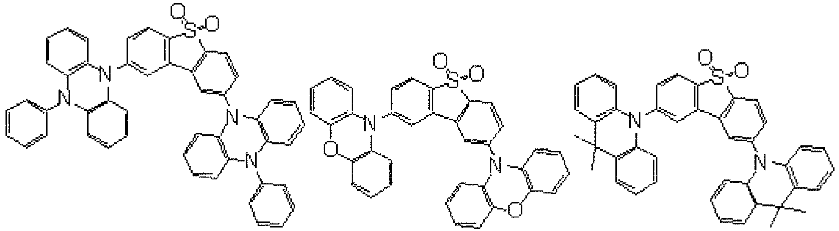

Only one kind of thermally activated delayed fluorescent light-emitting dopant material may be incorporated into the light-emitting layer, or two or more kinds of thermally activated delayed fluorescent light-emitting dopant materials may be incorporated into the layer. In addition, the thermally activated delayed fluorescent light-emitting dopant may be used after having been mixed with a phosphorescent light-emitting dopant or a fluorescent light-emitting dopant. When the layer contains two or more kinds of light-emitting dopants including the thermally activated delayed fluorescent light-emitting dopant material, the total weight of the light-emitting dopant materials is preferably 50% or less, more preferably 30% or less with respect to the host material.

When the light-emitting layer contains a light-emitting dopant, preferably when the layer contains a phosphorescent light-emitting dopant, a fluorescent light-emitting dopant, or a thermally activated delayed fluorescent light-emitting dopant, the material obtained by preliminarily mixing the two or more kinds of compounds each represented by the general formula (1) is used as the host material in the light-emitting layer, but any other compound except the compounds each represented by the general formula (1) may be further incorporated into the light-emitting layer for the purpose of adjusting a carrier balance in the layer. The other compound may be simultaneously preliminarily mixed with the compounds each represented by the general formula (1) in the preliminary mixing, but its preliminary mixing is not essential.

### -Injecting Layer-

The injecting layer refers to a layer formed between an electrode and an organic layer for the purposes of lowering a driving voltage and improving light emission luminance, and includes a hole-injecting layer and an electron-injecting layer. The injecting layer may be interposed between the anode and the light-emitting layer or the hole-transporting layer, or may be interposed between the cathode and the light-emitting layer or the electron-transporting layer. The injecting layer may be formed as required.

### -Hole-blocking Layer-

The hole-blocking layer has, in a broad sense, the function of an electron-transporting layer, and is formed of a hole-blocking material that has a remarkably small ability to transport holes while having a function of transporting electrons, and hence the hole-blocking layer is capable of improving the probability of recombining an electron and a hole in the light-emitting layer by blocking holes while transporting electrons.

A known material for a hole-blocking layer may be used for the hole-blocking layer, and a material for the electron-transporting layer to be described later may be used as required.

### -Electron-blocking Layer-

The electron-blocking layer has, in a broad sense, the function of a hole-transporting layer, and is capable of improving the probability of recombining an electron and a hole in the light-emitting layer by blocking electrons while transporting holes.

A known material for an electron-blocking layer may be used as a material for the electron-blocking layer, and a material for the hole-transporting layer to be described later may be used as required. The thickness of the electron-blocking layer is preferably from 3 nm to 100 nm, more preferably from 5 nm to 30 nm.

### -Exciton-blocking Layer-

The exciton-blocking layer refers to a layer for blocking excitons produced by the recombination of a hole and an electron in the light-emitting layer from diffusing into charge-transporting layers. The insertion of this layer enables efficient confinement of the excitons in the light-emitting layer, thereby being able to improve the luminous efficiency of the device. The exciton-blocking layer may be inserted on any of the anode side and the cathode side of the adjacent light-emitting layer, and may also be inserted simultaneously on both sides.

A known material for an exciton-blocking layer may be used as a material for the exciton-blocking layer. Examples thereof include 1,3-dicarbazolylbenzene (mCP) and bis(2-methyl-8-quinolinolato)-4-phenylphenolatoaluminum(III) (BAlq).

### -Hole-transporting Layer-

The hole-transporting layer is formed of a hole-transporting material having a function of transporting holes, and a single hole-transporting layer or a plurality of hole-transporting layers may be formed.

The hole-transporting material has a hole-injecting property or a hole-transporting property or has an electron-blocking property, and any of an organic material and an inorganic material maybeusedas the hole-transporting material. Anycompoundselected from conventionally known compounds may be used for the hole-transporting layer. Examples of such hole-transporting material include a porphyrin derivative, an arylamine derivative, a triazole derivative, an oxadiazole derivative, an imidazole derivative, a polyarylalkane derivative, a pyrazoline derivative, and a pyrazolone derivative, a phenylenediamine derivative, an arylamine derivative, an amino-substituted chalcone derivative, an oxazole derivative, a styrylanthracene derivative, a fluorenone derivative, a hydrazone derivative, a stilbene derivative, a silazane derivative, an aniline-based copolymer, and a conductive high-molecular weight oligomer, in particular, a thiophene oligomer. Of those, a porphyrin derivative, an arylamine derivative, or a styrylamine derivative is preferably used, and an arylamine compound is more preferably used.

### -Electron-transporting Layer-

The electron-transporting layer is formed of a material having a function of transporting electrons, and a single electron-transporting layer or a plurality of electron-transporting layers may be formed.

An electron-transporting material (which also serves as a hole-blocking material in some cases) only needs to have a function of transferring electrons injected from the cathode into the light-emitting layer. Any compound selected from conventionally known compounds may be used for the electron-transporting layer. Examples thereof include a nitro-substituted fluorene derivative, a diphenylquinone derivative, a thiopyran dioxide derivative, a carbodiimide, a fluorenylidenemethane derivative, anthraquinodimethane, an anthrone derivative, and an oxadiazole derivative. Further, it is also possible to use, as the electron-transporting material, a thiadiazole derivative prepared by substituting an oxygen atom on an oxadiazole ring with a sulfur atom in the oxadiazole derivative and a quinoxaline derivative that has a quinoxaline ring known as an electron withdrawing group. Further, it is also possible to use a polymer material in which any of those materials is introduced in a polymer chain or is used as a polymer main chain.

### Examples

The present invention is hereinafter described in more detail by way of Examples. The present invention is not limited to Examples below and may be carried out in various forms as long as the various forms do not deviate from the gist of the present invention.

The procedure described below was used to produce a preliminarily mixed host material. The number of each compound corresponds to the number given to the exemplified compound.

### Production of Preliminarily Mixed Host H1

Compound 2-13 (0.50g) and Compound 2 - 69 (0.50 g) were weighed, and were mixed while being ground in a mortar. Thus, a preliminarily mixed host H1 was produced.

### Production of Preliminarily Mixed Host H2

Compound 2-13 (0.50g) and Compound 2-59 (0.50 g) were weighed, and were mixed while being ground in a mortar. Thus, a preliminarily mixed host H2 was produced.

The evaporation temperatures of Compounds 2-13, 2-69, and 2-59 are shown in Table 1.

**Table 1**

| Compound | Evaporation temperature (°C) |
|---|---|
| 2-13 | 317 |
| 2-59 | 323 |
| 2-69 | 327 |

### Example 1

Each thin film was laminated on a glass substrate having formed thereon an anode formed of ITO having a thickness of 110 nm by a vacuum deposition method at a degree of vacuum of 4.0×10⁻⁵ Pa. First, CuPc serving as a hole-injecting layer was formed on ITO so as to have a thickness of 20 nm, and then NPB serving as a hole-transporting layer was formed so as to have a thickness of 20 nm. Next, the preliminarily mixed host H1 serving as a host for a light-emitting layer and Ir(PPy)₃ serving as a light-emitting dopant were co-deposited from different deposition sources to form a light-emitting layer having a thickness of 30 nm. At this time, a deposition rate ratio between the H1 and Ir (PPy) ₃ was 94:6. Next, BAlq serving as a hole-blocking layer was formed so as to have a thickness of 10 nm, and then Alq3 serving as an electron-transporting layer was formed so as to have a thickness of 40 nm. Further, lithium fluoride (LiF) serving as an electron-injecting layer was formed on the electron-transporting layer so as to have a thickness of 0.5 nm. Finally, aluminum (Al) serving as a cathode was formed on the electron-injecting layer so as to have a thickness of 100 nm. Thus, an organic EL device was produced.

An external power source was connected to the resultant organic EL device to apply a DC voltage to the device. As a result, an emission spectrum having a local maximum wavelength of 517 nm was observed, and hence it was found that light emission from Ir (PPy) ₃ was obtained. The luminance, driving voltage, luminous efficiency, and luminance half-life of the produced organic EL device are shown in Table 2. The luminance, the driving voltage, and the luminous efficiency are values at a driving current of 20 mA/cm², and are initial characteristics. The luminance half-life is a value at an initial luminance of 1,000 cd/m², and is a lifetime characteristic.

### Example 2

An organic EL device was produced in the same manner as in Example 1 except that in Example 1, the H2 was used as a host for a light-emitting layer. An external power source was connected to the resultant organic EL device to apply a DC voltage to the device. As a result, an emission spectrum having a local maximum wavelength of 517 nm was observed, and hence it was found that light emission from Ir(PPy)₃ was obtained. The luminance, driving voltage, luminous efficiency, and luminance half-life of the produced organic EL device are shown in Table 2.

### Comparative Example 1

An organic EL device was produced in the same manner as in Example 1 except that Compound 2-13 was used as a host for a light-emitting layer.

### Comparative Example 2

An organic EL device was produced in the same manner as in Example 1 except that Compound 2-69 was used as a host for a light-emitting layer.

### Comparative Example 3

As organic EL device was produced in the same manner as in Example 1 except that Compound 2-59 was used as a host for a light-emitting layer.

### Comparative Example 4

Am organic EL device was produced in the same manner as in Example 1 except that a preliminarily mixed host HC produced by weighing Compound A (0.50 g) below and Compound B (0.50 g) below, and mixing the compounds while grinding the compounds in a mortar was used as a host.

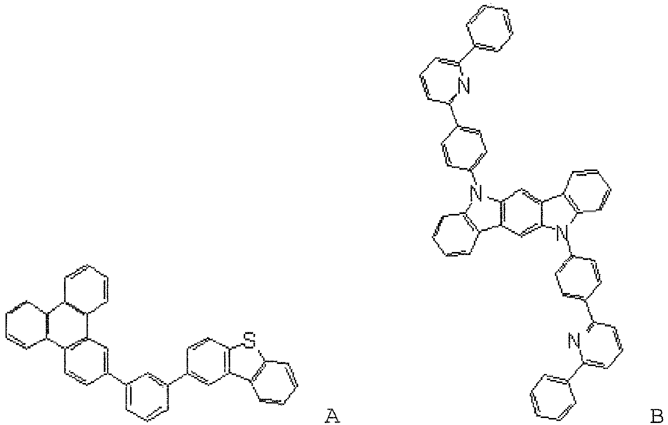

**Table 2**

| | Host | Luminance (cd/m²) | Voltage (V) | Luminous efficiency (lm/W) | Luminance half-life (h) |
|---|---|---|---|---|---|
| Example 1 | H1 | 6,870 | 6.8 | 15.9 | 32,890 |
| Example 2 | H2 | 6,950 | 7.0 | 15.6 | 29,860 |
| Comparative Example 1 | 2-13 | 6,530 | 6.3 | 16.3 | 15,380 |
| Comparative Example 2 | 2-59 | 2,060 | 9.2 | 3.5 | 2,570 |
| Comparative Example 3 | 2-69 | 1,230 | 9.8 | 2.0 | 1,380 |
| Comparative Example 4 | HC | 6,130 | 9.0 | 10.7 | 24,980 |

In Table 2, comparison between each of Examples 1 and 2 of the present invention, and each of Comparative Examples 1 to 3 shows that the use of a host material obtained by mixing compounds each represented by the general formula (1) improves light emission luminance and significantly lengthens a luminance half-life. In addition, comparison between Example 1 or 2 and Comparative Example 4 shows that Example 1 or 2 in which a host material obtained by mixing two kinds of compounds each represented by the general formula (1) is used is superior in both the initial luminance and the lifetime characteristic to Comparative Example 4.

### Examples 3 to 5

Organic EL devices were continuously produced three times in the same manner as in Example 1. The device produced in the first time was defined as Example 3, the device produced in the second time was defined as Example 4, and the device produced in the third time was defined as Example 5. The luminance, driving voltage, and luminous efficiency of each of the produced organic EL devices are shown in Table 3. The luminance, the driving voltage, and the luminous efficiency are values at a driving current of 20 mA/cm², and are initial characteristics.

### Comparative Examples 6 to 8

Each thin film was laminated on a glass substrate having formed thereon an anode formed of ITO having a thickness of 110 nm by a vacuum deposition method at a degree of vacuum of 4.0×10⁻⁵ Pa. First, CuPc serving as a hole-injecting layer was formed on ITO so as to have a thickness of 20 nm, and then NPB serving as a hole-transporting layer was formed so as to have a thickness of 20 nm. Next, as a light-emitting layer, Compound 2-13 serving as a host, Compound 2-59 serving as a secondhost, and Ir (PPy) ₃ serving as a light-emitting dopant were co-deposited from different deposition sources so as to have a thickness of 30 nm. At this time, a deposition rate ratio among the first host, the second host, and Ir(PPy)₃ was 47:47:6. Next, BAlq serving as a hole-blocking layer was formed so as to have a thickness of 10 nm. Then, Alq3 serving as an electron-transporting layer was formed so as to have a thickness of 40 nm. Further, lithium fluoride (LiF) serving as an electron-injecting layer was formed on the electron-transporting layer so as to have a thickness of 0.5 nm. Finally, aluminum (Al) serving as a cathode was formed on the electron-injecting layer so as to have a thickness of 100 nm. Thus, organic EL devices were continuously produced three times.

An external power source was connected to each of the resultant organic EL devices to apply a DC voltage to the device. As a result, an emission spectrum having a local maximum wavelength of 517 nm was observed, and hence it was found that light emission from Ir (PPy)₃ was obtained. The device produced in the first time was defined as Comparative Example 6, the device produced in the second time was defined as Comparative Example 7, and the device produced in the third time was defined as Comparative Example 8. The luminance, driving voltage, and luminous efficiency of each of the produced organic EL devices are shown in Table 3.

### Comparative Examples 9 to 11

Organic EL devices were continuously produced three times in the same manner as in Example 1 except that a preliminarily mixed host HD produced by weighing Compound 2-13 (0.50 g) and mCBP (0.50 g), and mixing the compounds while grinding the compounds in a mortar was used as a host. The device produced in the first time was defined as Comparative Example 9, the device produced in the second time was defined as Comparative Example 10, and the device produced in the third time was defined as Comparative Example 11. The luminance, driving voltage, and luminous efficiency of each of the produced organic EL devices are shown in Table 3. The evaporation temperature of mCBP is 288°C.

**Table 3**

| | Host | Second host | Luminance (cd/m²) | Voltage (V) | Luminous efficiency (lm/W) |
|---|---|---|---|---|---|
| Example 3 | H1 | | 6,820 | 6.8 | 15.7 |
| Example 4 | H1 | | 6.640 | 6.8 | 15.3 |
| Example 5 | H1 | | 6,780 | 6.7 | 15.9 |
| Comparative Example 6 | 2-13 | 2-69 | 6,660 | 6.7 | 15.6 |
| Comparative Example 7 | 2-13 | 2-69 | 6,120 | 6.7 | 14.3 |
| Comparative Example 8 | 2-13 | 2-69 | 6,230 | 7.0 | 14.0 |
| Comparative Example 9 | HD | | 6,540 | 6.2 | 16.6 |
| Comparative Example 10 | HD | | 6,020 | 6.6 | 14.3 |
| Comparative Example 11 | HD | | 3,470 | 8.9 | 6.1 |

In Table 3, comparison between each of Examples 3 to 5, and each of Comparative Examples 6 to 8 showed that the examples in each of which the preliminarily mixed host material was used were more excellent in initial characteristics, such as luminous efficiency, on average, and showed smaller variations in initial characteristics. In addition, it was found from the results of Comparative Examples 9 to 11 that variations in initial characteristics were large in the preliminarily mixed host HD containing the compounds whose evaporation temperatures differed from each other by more than 20°C.

It was revealed from those results that the use of a host material obtained by preliminarily mixing specific compounds was able to achieve an organic EL device showing high efficiency and a satisfactory lifetime characteristic with good reproducibility.

### Reference Signs List

substrate, 2 anode, 3 hole-injecting layer, 4 hole-transporting layer, 5 light-emitting layer, 6 electron-transporting layer, 7 cathode

## Claims

1. An organic electroluminescent device, comprising one or more light-emitting layers between an anode and a cathode opposite to each other, wherein at least one of the light-emitting layers produced by vacuum deposition contains a host material, which is obtained by preliminarily mixing two or more kinds of compounds selected from compounds each represented by the following general formula (1), and a light-emitting dopant material: 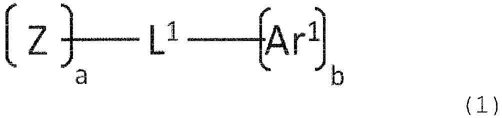 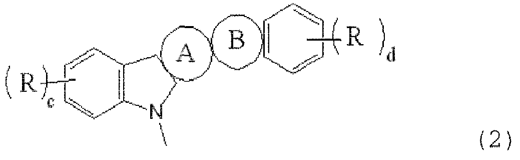 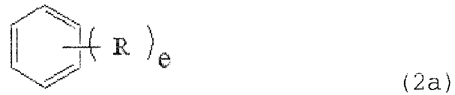 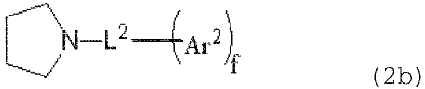 wherein,
Z represents an indolocarbazole ring-containing group represented by the general formula (2), aringArepresentsanaromatic hydrocarbon ring represented by the formula (2a), a ring B represents a heterocycle represented by the formula (2b), and the ring A and the ring B are each fused with an adjacent ring;
L¹ and L² each independently represent an aromatic hydrocarbon group having 6 to 30 carbon atoms, or an aromatic heterocyclic group having 3 to 18 carbon atoms, and Ar¹ and Ar² each independently represent an aromatic hydrocarbon group having 6 to 30 carbon atoms, an aromatic heterocyclic group having 3 to 18 carbon atoms, or a linked aromatic group obtained by linking 2 to 6 of the groups;
R's each independently represent a cyano group, an aliphatic hydrocarbon group having 1 to 10 carbon atoms, a diarylamino group having 12 to 44 carbon atoms, a diarylboryl group having 12 to 44 carbon atoms, an aromatic hydrocarbon group having 6 to 18 carbon atoms, or an aromatic heterocyclic group having 3 to 18 carbon atoms; and
a represents an integer of from 1 to 3 , b represents an integer of from 0 to 3, c and d each independently represent an integer of from 0 to 4, e represents an integer of from 0 to 2, and f represents an integer of from 0 to 3,
provided that L¹, L² Ar¹, Ar², and R's are each free from representing an indolocarbazole ring-containing group represented by the general formula (2).

2. An organic electroluminescent device according to claim 1, wherein at least one kind of the two or more kinds of compounds in the preliminarily mixed host material comprises a compound in which only one of L¹ and L² represents a nitrogen-containing aromatic heterocyclic group having 3 to 18 carbon atoms.

3. An organic electroluminescent device according to claim 1, wherein a difference in evaporation temperature between the two or more kinds of compounds in the preliminarily mixed host material is 20°C or less.

4. An organic electroluminescent device according to claim 1, wherein the light-emitting dopant material comprises a phosphorescent light-emitting dopant material formed of an organometallic complex containing at least one metal selected from ruthenium, rhodium, palladium, silver, rhenium, osmium, iridium, platinum, and gold.

5. An organic electroluminescent device according to claim 1, wherein the light-emitting dopant material comprises a fluorescent light-emitting dopant material.

6. An organic electroluminescent device according to claim 1, wherein the light-emitting dopant material comprises a thermally activated delayed fluorescent light-emitting dopant material.

7. A method of producing the organic electroluminescent device of claim 1, comprising depositing a material containing the host material, which is obtained by preliminarily mixing the two or more kinds of compounds selected from the compounds each represented by the general formula (1), and the light-emitting dopant material in a vacuum to produce at least one light-emitting layer.
